# FASCICULE DE BREVET EUROPEEN

(11) **EP 4 321 196 B1**
(45) Date de publication et mention de la délivrance du brevet: **07.05.2025**
(21) Numéro de dépôt: 23184088.5
(22) Date de dépôt: 07.07.2023
(51) Int. Cl.: A61M 16/00, A61M 16/12, A61M 16/16, A61M 16/20

(54) **INSTALLATION DE FOURNITURE DE GAZ COMPRENANT UN VENTILATEUR MÉDICAL ET UN DISPOSITIF DE DÉLIVRANCE DE NO AVEC UN SYSTÈME DE DOSAGE D'URGENCE**
GASVERSORGUNGSANLAGE MIT EINEM MEDIZINISCHEN BEATMUNGSGERÄT UND EINER NO-ABGABEVORRICHTUNG MIT EINEM NOTFALLDOSIERSYSTEM
GAS SUPPLY SYSTEM COMPRISING A MEDICAL VENTILATOR AND A NO-DELIVERY DEVICE WITH AN EMERGENCY METERING SYSTEM

(30) Priorité: 11.08.2022 FR 2208243
(43) Date de publication de la demande: 14.02.2024
(73) Titulaire: INOSYSTEMS, 92160 Antony (FR)
(72) Inventeur: BOULANGER, Thierry, 92160 Antony (FR); MARCHAL, Frédéric, 92160 Antony (FR); SCHMITT, Mary, 92200 Bagneux (FR)
(74) Mandataire: Air Liquide

(56) Documents cités:
- EP-A1- 2 522 384
- EP-A1- 3 888 727
- WO-A1-2016/096056

## Description

L'invention concerne une installation de fourniture de gaz comprenant un ventilateur médical, c'est-à-dire un appareil médical d'administration de gaz, et un dispositif ou appareil de délivrance de monoxyde d'azote gazeux (NO) pour fournir un gaz contenant du NO à un patient, via un système de délivrance principal de NO, laquelle installation comprend en outre un système de dosage d'urgence de NO permettant de fournir le gaz contenant du NO à un débit de secours donné en cas de dysfonctionnement du système de délivrance principal de NO, en particulier en cas de perte du signal provenant du capteur de débit agencé sur le circuit patient alimenté par le ventilateur médical.

Le NO est un gaz qui, lorsqu'inhalé, dilate les vaisseaux pulmonaires et augmente l'oxygénation en améliorant les échanges gazeux. Les propriétés du NO sont utilisées pour traiter différentes conditions médicales, comme l'Hypertension Artérielle Pulmonaire du nouveau-né ou PPHN (pour *Persistent Pulmonary Hypertension of the Newborn*)*,* le Syndrome de Détresse Respiratoire Aigüe ou SDRA observé principalement chez l'adulte ou encore les hypertensions pulmonaires en chirurgie cardiaque, comme enseigné notamment par EP-A-560928, EP-A-1516639 ou US-A-10,201,564.

Usuellement, une faible quantité de NO gazeux (i.e. quelques ppm vol.), dilué dans de l'azote (N₂) est injecté dans un flux gazeux contenant au moins 21% vol. d'oxygène (O₂) qui est ensuite inhalé par le patient. La concentration de NO, qui correspond à une posologie, est déterminée par le médecin ou analogue. Typiquement, le gaz contenant l'O₂ est typiquement un mélange N₂/O₂ ou de l'air, tel de l'air de qualité médical. En général, la concentration ou posologie de NO dans le gaz inhalé par le patient, c'est-à-dire après mélange du mélange NO/N₂ avec l'air ou un mélange O₂/N₂, est comprise entre 1 et 80 ppm en volume (ppmv), en fonction de la population traitée, i.e. nouveau-nés ou adultes, et donc de la maladie à traiter.

Le gaz inhalé par le patient peut être délivré par le biais d'un dispositif de délivrance de NO associé à un ventilateur mécanique, comme décrit par US-A-5,558,083. Le dispositif de délivrance de NO est fluidiquement connecté à une ou plusieurs bouteilles de gaz contenant un mélange de N₂/NO dont la concentration en NO comprise typiquement entre 200 et 800 ppmv. Généralement, le système de délivrance de NO comprend un module d'injection de NO placé dans la branche inspiratoire d'un circuit patient connecté fluidiquement, d'une part, au ventilateur mécanique et, d'autre part, à une interface respiratoire délivrant le gaz enrichi en NO au patient, par exemple un masque respiratoire, une sonde d'intubation trachéale ou similaire.

Une installation de délivrance de NO comprend également un capteur de débit qui mesure le débit gazeux délivré par le ventilateur mécanique (i.e. air ou mélange N₂/O₂) dans le circuit patient afin de déterminer la quantité de NO à délivrer pour respecter la posologie fixée par le médecin. Il est à noter que ce capteur dit « de débit » peut être du type mesurant et fournissant des signaux ou mesures de débit proprement-dits mais aussi du type mesurant et fournissant des signaux ou mesures de pression qui sont converties ensuite en débit par des moyens de pilotage, en particulier au sein d'un microprocesseur.

Le dosage en NO peut être assuré par le biais d'une électrovanne proportionnelle délivrant un flux continu de gaz contenant le NO, laquelle est associée à un capteur de débit, dit capteur de débit de NO, ces deux composants étant agencés dans le dispositif de délivrance de NO, ainsi que par une ligne d'injection reliant le dispositif de délivrance de NO au module d'injection de NO agencé sur le circuit patient, tel que décrit dans US-A-5,558,083.

D'autres dispositifs de délivrance de NO existent, dans lesquels l'électrovanne proportionnelle est remplacée par une pluralité d'électrovannes de type « tout ou rien », délivrant le gaz de façon intermittente, c'est-à-dire sous forme de pulses, généralement à haute fréquence, dont l'amplitude et la durée permettent de garantir la bonne quantité de gaz circulant dans la ligne d'injection reliée au module d'injection de NO.

Dans tous les cas, les installations de délivrance de NO connus reçoivent les mesures ou signaux du capteur de débit placé dans la branche inspiratoire du circuit patient et ajustent en temps réel la quantité de NO devant être délivrée, selon la posologie désirée, en contrôlant le flux de NO dans la ligne d'injection. L'association du capteur de débit placé dans la branche inspiratoire du circuit patient et la ou les électrovannes proportionnelles ou de type « tout ou rien » et le capteur de débit de NO, si présent, ainsi que les moyens de pilotages comme décrit après qui sont agencés dans le dispositif de délivrance de NO, forment un système de délivrance principal de NO.

Le NO étant un agent thérapeutique efficace, c'est-à-dire que de très faibles concentrations (i.e. quelques ppmv) produisent un effet thérapeutique, son juste dosage est d'importance critique et les équipes médicales doivent constamment adapter la posologie selon l'état du patient. Dès lors, lorsque l'état du patient évolue, la concentration de NO doit être progressivement diminuée ou, à l'inverse, augmentée pour tenir compte de l'état du patient. Par exemple, dans une situation de sevrage du nouveau-né dont l'état s'améliore, il est usuel de décroitre progressivement la posologie, par exemple par pas de 1 ppm, jusqu'à atteindre une valeur nulle permettant alors de stopper la délivrance de NO. Une diminution progressive de la concentration en NO permet d'éviter l'« effet rebond » pouvant se manifester en cas de variation rapide de la concentration, typiquement en cas de discontinuation brutale du traitement, qui pourrait avoir pour effet d'empirer gravement l'état du patient.

Or, les installations de délivrance de NO sont des systèmes électro-médicaux sophistiqués susceptibles de subir des défaillances, c'est-à-dire des dysfonctionnements, pouvant avoir un impact important sur la thérapie en cours. Par exemple, un dysfonctionnement ou défaut électronique, en particulier des moyens de pilotage et/ou du système de délivrance principal de NO, peut entrainer une panne de l'appareil et donc un arrêt total de délivrance de NO, avec les conséquences négatives susmentionnées.

Les défaillances possibles sont multiples comme par exemple :
- une perte du signal provenant du capteur de débit mesurant le débit gazeux délivré par le ventilateur mécanique, lequel capteur de débit est agencé dans le circuit patient du ventilateur et soumis à des contraintes multiples de la part des utilisateurs ;
- une perte de connexion électrique entre la ou les électrovannes ou bien le capteur de débit de NO et les moyens de pilotage, suite à des vibrations répétées se manifestant par exemple lors du transport d'un patient ; ou
- une perte brutale des moyens de pilotages en cas de défaillance majeure, provenant par exemple d'un dysfonctionnement ou défaut électronique.

Dans de telles circonstances, le dispositif de délivrance de NO doit avertir l'utilisateur au moyen d'un signal d'alarme audible et/ou visuel qu'une action rapide est requise, par exemple de basculer sur un mode d'injection pneumatique de secours afin de limiter autant que possible, les effets indésirables liés à une discontinuation de la thérapie engendrée par la défaillance.

Un tel basculement en mode de secours se fait habituellement par actionnement manuel d'un bouton rotatif ou analogue commandant le passage du mode d'administration normal de NO vers un mode de secours, dans lequel est par exemple opérée une délivrance continue d'un débit fixe de mélange N₂/NO, i.e. de l'ordre de 250 mL/min. Or, un tel mécanisme ou système de dosage de secours n'est pas sans risque, notamment pour les raisons suivantes :
- son activation requiert la présence d'une personne ayant autorité pour entreprendre cette action, par exemple un médecin en néonatologie. Il peut ainsi se passer plusieurs minutes avant que cette personne arrive et donc que le dosage de secours ne soit établi, ce qui entraine une discontinuation de la thérapie et expose le patient à un effet rebond ;
- le dosage de secours, i.e. un débit unique de mélange N₂/NO, ne permet pas de garantir que la posologie désirée soit respectée. En particulier, quand le dosage de secours est très inférieur à la posologie souhaitée, le patient peut être exposé à un changement abrupte de concentration et potentiellement sujet à d'importants effets indésirables ; et/ou
- le dosage de secours est incompatible avec certains types de ventilateurs délivrant de très faibles volumes, tels que les ventilateurs à oscillations hautes fréquence (HFO) car il peut en résulter une concentration en NO inhalée trop élevée pouvant atteindre des niveaux dangereux pour le patient. Dès lors, en cas de traitement du patient avec un tel ventilateur (i.e. HFO), on se retrouve alors sans moyens d'administrer le NO au patient, ce qui entraine les risques susmentionnés liés à l'arrêt brusque de traitement.

Il apparaît dès lors que les mécanismes de dosage de secours actuels ne permettant pas de garantir un niveau satisfaisant de sécurité et qu'il serait souhaitable pour le patient, en cas de mise en place d'un dosage de secours du fait d'un dysfonctionnement de l'installation de délivrance de NO, de pouvoir maintenir une thérapie par NO, sans commettre d'interruption de la thérapie, et sans se soucier du type de ventilateur, i.e. HFO ou autre, avec lequel coopère le système de délivrance de NO de l'installation de délivrance de NO.

Pour tenter d'y remédier, EP-A-3410927 propose un dosage en réponse à la perte du signal, i.e. des données, provenant du capteur de débit mesurant le débit gazeux délivré par le ventilateur mécanique, lequel capteur de débit est agencé dans le circuit patient du ventilateur et soumis à des contraintes multiples de la part des utilisateurs. La solution proposée repose sur un pilotage de l'électrovanne proportionnelle du dispositif de délivrance de NO de manière à délivrer un débit de NO constant basé sur des données historiques stockées ayant été précédemment mesurées par le capteur de débit mesurant le débit gazeux délivré par le ventilateur et stockées dans une mémoire de l'appareil.

Toutefois, cette solution est imparfaite car elle ne permet pas d'assurer la délivrance d'un débit de NO moyen en cas de défaillance autre, comme une perte de connexion électrique avec la ou les électrovannes, ou entre le capteur de débit de NO et les moyens de pilotage, ou encore une perte brutale des moyens de pilotage eux-mêmes.

WO2016096056A1 propose un système de secours avec un valeur de dosage constante prédéfinie dans l'unité de contrôle. Autrement dit, un problème est de pouvoir maintenir une posologie, c'est-à-dire un traitement du patient par NO inhalé à la concentration désirée, en cas de défaillance ou dysfonctionnement du capteur de débit qui est typiquement situé dans le circuit patient et auquel sont raccordés le ventilateur médical et le dispositif de délivrance de NO mais aussi des défaillances autres comme susmentionné.

Une solution selon l'invention concerne une installation de fourniture de gaz à un patient comprenant :
- un dispositif ou appareil de délivrance de NO (i.e. de fourniture de NO) configuré pour fournir un mélange gazeux NO/N₂ comprenant :
   ▪ une ligne d'injection de NO pour acheminer le mélange gazeux NO/N₂,
   ▪ un dispositif à vanne agencé sur la ligne d'injection pour contrôler la circulation du mélange gazeux NO/N₂ dans la ligne d'injection,
   ▪ une ligne de secours venant se raccorder fluidiquement à la ligne d'injection en amont et en aval du dispositif à vanne, ladite ligne de secours comprenant une électrovanne de secours et un dispositif de contrôle de débit, et
   ▪ des moyens de pilotage, aussi appelés unité de pilotage, configurés pour coopérer avec l'électrovanne de secours, le dispositif de contrôle de débit et le dispositif à vanne,
- un ventilateur médical configuré pour fournir un gaz respiratoire contenant de l'oxygène, typiquement environ au moins 21% d'oxygène, tel de l'air ou un mélange N₂/O₂,
- un circuit patient auquel sont raccordés fluidiquement le dispositif de délivrance de NO et le ventilateur médical pour alimenter ledit circuit patient en ledit mélange gazeux NO/N₂ et en ledit gaz respiratoire contenant de l'oxygène, typiquement environ au moins 21% d'oxygène, et
- un capteur de débit configuré pour déterminer (i.e. mesurer) et fournir aux moyens de pilotage du dispositif de délivrance de NO, au moins un signal de mesure représentatif du débit gazeux au sein du circuit patient, à savoir un (ou des) signal ou une(ou des) mesure de débit ou de pression.

De plus, selon l'invention, en cas d'interruption de la réception, c'est-à-dire en cas de perte de signal, par les moyens de pilotage du dispositif ou appareil de délivrance de NO, du signal de mesure fourni par le capteur de débit :
- l'électrovanne de secours passe, i.e. est configurée pour passer, en position ouverte pour permettre une circulation du mélange gazeux NO/N₂ dans la ligne de secours,
- le dispositif à vanne passe, i.e. est configurée pour passer, en position fermée pour stopper toute circulation de gaz dans la ligne d'injection, et
- le dispositif de contrôle de débit est configuré pour fournir le mélange gazeux NO/N₂ à un débit gazeux de secours préfixé, i.e. préréglé, où ledit débit gazeux de secours est déterminé par les moyens de pilotage à partir d'au moins un signal de mesure fourni par le capteur de débit, avant ladite interruption de réception dudit signal, et préréglé par commande du dispositif de contrôle de débit par lesdits moyens de pilotage, avant ladite interruption de réception dudit signal.

Dans le cadre de l'invention :
- « ppmv » signifie partie par million en volume,
- « %vol. » » signifie pourcentage en volume.
- « NO » désigne le monoxyde d'azote.
- « N₂ » désigne l'azote.
- « O₂ » désigne l'oxygène.
- Par « fonctionnement normal » : on entend un fonctionnement habituel, en l'absence de toute panne, dysfonctionnement, défaut ou autre.
- Par « dysfonctionnement », on entend une panne, un défaut, une déconnexion ou analogue, réversible ou définitif, d'origine électrique, mécanique ou d'une autre nature, affectant le fonctionnement normal en empêchant toute réception par les moyens de pilotage, du ou des signaux mesurés (i.e. des mesures opérées) et transmis par le capteur de débit.
- Par « capteur de débit », on entend un capteur du type mesurant et fournissant un ou des signaux ou mesures de débit proprement-dits ou du type mesurant et fournissant un ou des signaux ou mesures de pression qui sont converties ensuite en débit par les moyens de pilotage.

Selon le mode de réalisation considéré, le dispositif ou appareil de délivrance de NO et/ou l'installation de l'invention peuvent comprendre l'une ou plusieurs des caractéristiques suivantes :
- le dispositif à vanne du dispositif de délivrance de NO est configuré pour être normalement dans une position fermée (i.e. état repos) pour empêcher, i.e. stopper ou s'opposer à, toute circulation de gaz dans la ligne d'injection, c'est-à-dire que la position fermée correspond à son état de repos.
- l'électrovanne de secours du dispositif de délivrance de NO est configurée pour être normalement dans une position ouverte (i.e. état de repos) pour permettre, i.e. autoriser, une circulation de gaz dans la ligne de secours, c'est-à-dire que la position ouverte correspond à son état de repos.
- dans leur état de repos, le dispositif à vanne et l'électrovanne de secours du dispositif de délivrance de NO ne sont plus ou pas commandés par les moyens de pilotage.
- en cas de perte de signal, les moyens de pilotage sont configurés pour arrêter de piloter (i.e. ne plus commander) le dispositif à vanne et l'électrovanne de secours du dispositif de délivrance de NO qui passent alors automatiquement dans leur état de repos.
- en cas de perte de signal, les moyens de pilotage sont configurés pour arrêter de piloter (i.e. ne plus commander) le dispositif à vanne et l'électrovanne de secours du dispositif de délivrance de NO de sorte que :
   ∘ ledit dispositif à vanne passe d'une position ouverte autorisant le passage de gaz dans la ligne d'injection à la position fermée et
   ∘ ladite électrovanne de secours passe d'une position fermée empêchant le passage de gaz dans la ligne de secours à la position ouverte.
- un dispositif de mesure de débit est agencé sur la ligne d'injection du dispositif de délivrance de NO pour opérer une ou des mesures de débit du gaz contenant du NO circulant dans la ligne d'injection.
- la ligne de secours est raccordée fluidiquement à la ligne d'injection en amont ou en aval du dispositif de contrôle de débit.
- les moyens de pilotage (i.e. dispositif de pilotage) sont en outre configurés pour calculer le débit gazeux de secours (i.e. débit de mélange NO/N₂ de secours) à partir du dernier débit de gaz mesuré par le capteur de débit mesurant le débit de gaz provenant du ventilateur, avant ladite interruption de réception dudit signal.
- les moyens de pilotage sont en outre configurés pour déterminer, i.e. calculer, le débit de secours à partir d'un ou plusieurs débits de gaz respiratoire, de préférence plusieurs débits de gaz, fournis par le ventilateur ayant été mesuré par le capteur de débit, avant ladite interruption de réception dudit signal, et d'une posologie souhaitée, c'est-à-dire une concentration de NO finale à obtenir après mélange du mélange NO/azote provenant du dispositif de délivrance de NO et du flux de gaz respiratoire contenant au moins 21% d'O₂ provenant du ventilateur, typiquement de l'air ou un mélange oxygène/azote.
- le dispositif de contrôle de débit est configuré pour fournir le mélange gazeux NO/N₂ à un débit gazeux de secours préfixé, déterminé par les moyens de pilotage à partir de plusieurs signaux de mesure fournis par le capteur de débit pendant une durée donnée, avant ladite interruption de réception dudit signal, par exemple pendant plusieurs secondes ou dizaines de secondes, lesdits signaux de mesure étant utilisés pour calculer un débit moyen sur ladite durée donnée.
- le débit gazeux de secours est déterminé par les moyens de pilotage à partir d'un débit de gaz moyen calculé à partir de plusieurs débits de gaz mesurés par le capteur de débit pendant une durée donnée, avant ladite interruption de réception dudit signal, de préférence la durée donnée est comprise entre plusieurs secondes et plusieurs dizaines de secondes.
- les moyens de pilotage sont configurés pour déterminer, i.e. calculer, le débit de secours à partir d'un ou de préférence plusieurs débits de gaz respiratoire fournis par le ventilateur ayant été mesurés par le capteur de débit, avant ladite interruption de réception dudit signal, et d'une posologie souhaitée où la teneur en NO dans le mélange administré au patient est comprise entre 1 et 80 ppmv.
- les moyens de pilotage du dispositif de délivrance de NO sont en outre configurés pour, pendant le fonctionnement normal (i.e. avant tout dysfonctionnement), commander l'électrovanne de secours pour qu'elle soit en une position fermée (i.e. état actif) empêchant toute circulation de gaz dans la ligne de secours.
- les moyens de pilotage du dispositif de délivrance de NO sont en outre configurés pour, pendant le fonctionnement normal, commander le dispositif à vanne pour autoriser (i.e. état actif) une circulation de gaz dans la ligne d'injection et au moins une mesure de débit de gaz par le dispositif de mesure de débit.
- les moyens de pilotage sont en outre configurés pour contrôler, avant tout dysfonctionnement avec perte de signal provenant du capteur de débit (i.e. pendant le fonctionnement normal), le dispositif de contrôle de débit de la ligne de secours pour pré-régler le débit gazeux de secours à délivrer en cas d'interruption de réception de signal du capteur de débit, c'est-à-dire en cas de perte de signal provenant du capteur de débit.
- pendant le fonctionnement normal, le capteur de débit est configuré pour opérer plusieurs mesures de débit ou de pression (i.e. du gaz respiratoire fourni par le ventilateur) et transmettre la ou lesdites mesures de débit ou de pression aux moyens de pilotage, de préférence via un capteur de pression différentiel.
- pendant le fonctionnement normal, les moyens de pilotage sont en outre configurés pour déterminer, i.e. calculer, un débit gazeux moyen pendant une durée donnée à partir de plusieurs signaux ou mesures de débit ou de pression opérées par le capteur de débit (i.e. du gaz respiratoire fourni par le ventilateur), c'est-à-dire le débit de gaz respiratoire fourni par le ventilateur moyenné sur la durée donnée, par exemple sur plusieurs secondes ou dizaines de secondes.
- le débit moyen est calculé par les moyens de pilotage sur une durée donnée de plusieurs secondes à plusieurs dizaines de secondes, typiquement entre 5 et 60 secondes, ou une autre durée.
- les moyens de pilotage sont en outre configurés pour mémoriser au moins un débit gazeux (i.e. du gaz respiratoire fourni par le ventilateur), notamment le débit moyen, déterminé à partir du ou des signaux ou mesures de débit ou de pression provenant du capteur de débit (i.e. du gaz respiratoire fourni par le ventilateur).
- le gaz respiratoire contenant de l'oxygène fourni par le ventilateur est de l'air ou un mélange azote/oxygène contenant environ au moins 21%vol. d'oxygène, voire de l'oxygène pur (i.e. env. 100%vol.).
- il comprend des moyens de mémorisation.
- les valeurs de débit obtenues via le capteur de débit sont mémorisées dans les moyens de mémorisation du dispositif de délivrance de NO.
- les moyens de mémorisation comprennent une mémoire informatique, par exemple une mémoire vive.
- le circuit patient comprend un module d'injection de NO alimenté en mélange NO/N₂ par le dispositif de délivrance de NO, de préférence via la ligne d'injection, telle une conduite de gaz flexible.
- le capteur de débit est agencé sur le circuit patient, de préférence sur une branche inspiratoire dudit circuit patient, et est relié électriquement aux moyens de pilotage, via une ou de liaisons électriques (e.g. câbles ou analogues).
- le capteur de débit (i.e. servant à mesurer le débit du gaz respiratoire fourni par le ventilateur) est agencé sur le circuit patient entre le ventilateur médical et le module d'injection de NO.
- selon un mode réalisation, le capteur de débit est du type capteur de débit massique ou analogue. Dans ce cas, le capteur de débit est relié électriquement aux moyens de pilotage, via une ou des liaisons électriques, tels des câbles ou analogues.
- selon un autre mode réalisation, le capteur de débit est du type à mesure de pression différentielle, c'est-à-dire un capteur de pression. Dans ce cas, le capteur de débit est agencé sur le circuit patient et coopère avec un capteur de pression différentiel agencé dans l'appareil de délivrance de NO.
- le capteur de pression différentiel est agencé sur une carte électronique dans l'appareil de délivrance de NO et coopère avec les moyens de pilotage de l'appareil de délivrance de NO, de préférence une même carte électronique porte ledit capteur de pression différentiel et au moins un microprocesseur desdits moyens de pilotage.
- le capteur de débit comprend un module de mesure de pression agencé sur le circuit patient, en particulier dans la branche inspiratoire, comprenant des prises de mesure de pression.
- le module de mesure est traversé par le flux gazeux circulant dans le circuit patient, en particulier dans la branche inspiratoire.
- les prises de mesure de pression du module de mesure du capteur de débit situées sur le circuit patient sont reliées pneumatiquement au capteur de pression différentiel par une ligne amont et la ligne aval de mesure de pression afin de fournir des pressions de gaz audit capteur de pression différentiel. De préférence, la ligne amont et la ligne aval sont des conduits de gaz, des tubulures ou analogue.
- l'interruption de la réception, par les moyens de pilotage du dispositif de délivrance de NO, dudit au moins un signal de mesure fourni par le capteur de débit résulte, i.e. est consécutif, d'un débranchement intempestif d'au moins l'une des lignes amont et aval de mesure de pression.
- le module de mesure de pression du capteur de débit comprend un passage interne de gaz comprenant une restriction interne créant une perte de charge, i.e. engendrant un différentiel ou gradient de pression, lorsqu'un débit gazeux circule dans le passage interne et traverse ladite restriction interne.
- le module de mesure de pression du capteur de débit comprend des chambres amont et aval, au sein du passage interne du capteur de débit, séparées par une paroi traversée un orifice de passage de gaz de sorte de former la restriction interne.
- la ligne amont et la ligne aval de mesure de pression relient fluidiquement le module de mesure de pression du capteur de débit au capteur de pression différentiel de manière à permettre de mesurer la pression avant et après perte de charge, c'est-à-dire en en amont et en aval de la restriction interne.
- la ligne amont et la ligne aval de mesure de pression sont connectées fluidiquement aux chambres amont et aval du module de mesure de pression du capteur de débit afin d'y opérer des mesures de pression du flux circulant, c'est-à-dire avant et après perte de charge.
- la ligne amont et la ligne aval de mesure de pression comprennent des conduits de mesure de pression ou analogues configurés pour fournir au capteur de pression différentiel, les pressions mesurées avant et après perte de charge.
- le capteur de pression différentiel est agencé dans le dispositif de délivrance de NO.
- le capteur de pression différentiel est connecté électriquement aux moyens de pilotage ou est configuré pour transmettre les mesures de pression provenant du capteur de débit, aux moyens de pilotage.
- les moyens de pilotage sont configurés pour traiter informatiquement les mesures de pression, i.e. différentiel de pression, et en déduire au moins un débit de gaz.
- les moyens de pilotage sont configurés pour déterminer au moins un débit de gaz respiratoire provenant du ventilateur et circulant dans la branche inspiratoire à partir des valeurs de pression et d'au moins une table de correspondance préenregistrée permettant de transformer une (les) valeur de pression, notamment transmise par le capteur de pression différentiel, en valeur de débit.
- la (ou les) table de correspondance préenregistrée est enregistrée dans les moyens de mémorisation.
- le capteur de pression différentiel est agencé dans le dispositif de délivrance de NO et coopère avec les moyens de pilotage pour leur fournir les valeurs de pression mesurées par le module de mesure du capteur de débit.
- la ligne de secours du dispositif de délivrance de NO vient se raccorder fluidiquement à la ligne d'injection par une extrémité amont, en amont du dispositif à vanne, et par une extrémité aval, en aval du dispositif à vanne de manière à bipasser ledit dispositif à vanne.
- le dispositif de délivrance de NO comprend un système de dosage de secours de NO comprenant la ligne de secours.
- le dispositif de mesure de débit du dispositif de délivrance de NO est agencé sur la ligne d'injection en amont ou en aval du dispositif à vanne, de préférence en aval du dispositif à vanne.
- la ligne d'acheminement de NO du dispositif de délivrance de NO achemine un mélange gazeux formé de NO et d'azote, de préférence un mélange gazeux NO/N₂ contenant entre 100 et 2000 ppmv de NO, typiquement moins de 1000 ppmv de NO, le reste étant de l'azote (et éventuellement des impuretés inévitables)
- l'électrovanne de secours du dispositif de délivrance de NO est configurée et/ou pilotée pour être normalement ouverte.
- l'électrovanne de secours du dispositif de délivrance de NO est du type tout ou rien.
- les moyens de pilotage du dispositif de délivrance de NO comprennent au moins un microprocesseur.
- les moyens de pilotage du dispositif de délivrance de NO comprennent une carte électronique portant ledit au moins un microprocesseur.
- la ligne d'injection du dispositif de délivrance de NO est raccordée fluidiquement à une ligne haute pression par l'intermédiaire d'un dispositif régulateur de pression, la ligne haute pression et le dispositif régulateur de pression étant agencés dans le dispositif de délivrance de NO.
- le dispositif de délivrance de NO comprend un boitier.
- le système de dosage de secours de NO est agencé dans le boitier du dispositif de délivrance de NO, en particulier la ligne de secours et l'électrovanne de secours.
- le dispositif à vanne du dispositif de délivrance de NO comprend une électrovanne, de préférence une électrovanne proportionnelle.
- le dispositif de contrôle de débit du dispositif de délivrance de NO est configuré pour former, constituer ou comprendre un orifice calibré de type proportionnel, c'est-à-dire former un système à orifice calibré proportionnel.
- le dispositif de contrôle de débit comprend un système à orifice calibré proportionnel commandé par les moyens de pilotage pour régler le débit gazeux de secours préfixé.
- les moyens de pilotage du dispositif de délivrance de NO sont en outre configurés pour, préalablement à toute interruption de la réception ou perte du (des) signal provenant du capteur de débit, agir sur le système à orifice calibré proportionnel du dispositif de contrôle de débit de la ligne de secours pour prérégler (i.e. régler ou ajuster avant toute perte de signal) le débit gazeux de secours désiré, i.e. débit de NO/N₂ de secours, lequel est déterminé à partir du débit de gaz respiratoire ayant été mesuré par le capteur de débit, avant l'interruption de réception dudit signal, i.e. perte de signal, et de la posologie en NO souhaitée.
- le dispositif de contrôle de débit du dispositif de délivrance de NO comprend un moyen actionneur coopérant avec un élément mobile comprenant un évidement traversant, ledit élément mobile pouvant être déplacé angulairement par le moyen actionneur.
- le moyen actionneur comprend un moteur électrique entrainant un axe rotatif, l'élément mobile étant solidaire dudit axe rotatif.
- le moteur électrique est un moteur pas à pas.
- l'élément mobile est agencé mobile dans un logement interne comprenant un port d'entrée et un port de sortie en communication fluidique avec la ligne de secours.
- l'élément mobile est une sphère, c'est-à-dire sphérique, par exemple une bille ou analogue.
- le logement interne a une forme sphérique complémentaire de celle de l'élément mobile sphérique.
- les moyens de pilotage sont configurés pour piloter le moyen actionneur pour opérer un déplacement angulaire de l'élément mobile entre au moins :
   ∘ une position d'ouverture totale correspondant à un niveau d'ouverture totale de l'orifice calibré du dispositif de contrôle de débit,
   ∘ une position de fermeture totale correspondant à un niveau de fermeture (i.e. obturation) totale de l'orifice calibré du dispositif de contrôle de débit, et
   ∘ au moins une position intermédiaire située entre lesdites position d'ouverture totale et position de fermeture totale correspondant à un niveau d'ouverture partiel de l'orifice calibré du dispositif de contrôle de débit.
- les moyens de pilotage du dispositif de délivrance de NO sont configurés pour piloter le moyen actionneur pour opérer un déplacement angulaire de l'élément mobile entre au moins :
   ∘ une position d'ouverture totale dans laquelle tout le débit gazeux amené par la ligne de secours pénètre dans l'évidement traversant de l'élément mobile, c'est-à-dire un débit maximal,
   ∘ une position de fermeture totale dans laquelle aucun débit gazeux ne peut traverser l'évidement traversant de l'élément mobile, c'est-à-dire un débit nul, et
   ∘ au moins une position intermédiaire située entre lesdites position d'ouverture totale et position de fermeture totale, dans laquelle seulement une partie du débit gazeux amené par la ligne de secours pénètre dans l'évidement traversant de l'élément mobile, c'est-à-dire un ou des débits réduits.
- les moyens de pilotage du dispositif de délivrance de NO sont configurés pour piloter le moyen actionneur pour opérer un déplacement angulaire de l'élément mobile entre plusieurs positions intermédiaires, angulairement décalées les uns des autres, correspondant chacune à un niveau d'ouverture d'orifice calibré et/ou à un débit de gaz donné, c'est-à-dire des débits réduits compris entre le débit maximal et le débit nul.
- les moyens de pilotage du dispositif de délivrance de NO sont configurés pour piloter le moyen actionneur pour régler ou ajuster le débit gazeux de secours, avant toute perte de signal provenant du capteur de débit.
- les moyens de pilotage du dispositif de délivrance de NO sont configurés pour déterminer une ouverture d'orifice calibré donné correspondant au débit de secours.
- les moyens de pilotage du dispositif de délivrance de NO sont configurés pour déterminer l'ouverture d'orifice calibré donné et/ou le débit de secours à partir d'une table de correspondance.
- le dispositif de délivrance de NO comprend des moyens d'alimentation électrique alimentant en courant électrique les composants nécessitant de l'énergie électrique pour fonctionner, notamment les moyens de pilotage.
- les moyens d'alimentation électrique comprend des moyens de raccordement au secteur (110/220V) et/ou une batterie ou analogue.
- le débit gazeux de secours est déterminée par les moyens de pilotage à partir de la ou des dernières mesures de débit du flux de gaz respiratoire (i.e. air ou N₂/O₂) circulant dans la branche inspiratoire, ayant été opérée par le capteur de débit, avant le dysfonctionnement (i.e. perte de signal), et transmise aux moyens de pilotage du dispositif de délivrance de NO. Les moyens de pilotage utilisent en outre la posologie de NO souhaitée dans le mélange gazeux final, typiquement entre 1 et 80 ppmv pour déterminer le débit gazeux de secours.

De plus, l'installation de fourniture de gaz à un patient comprend en outre :
- au moins une source de NO contenant un mélange gazeux NO/N₂ et alimentant en ledit mélange gazeux NO/N₂, le dispositif de délivrance de NO.
- un circuit patient comprenant une branche inspiratoire alimentée en mélange gazeux NO/N₂ par le dispositif de délivrance de NO, et en un gaz respiratoire contenant de l'oxygène, typiquement environ au moins 21% d'oxygène, tel de l'air ou un mélange O₂/N₂, par le ventilateur médical, i.e. un appareil d'assistance respiratoire.
- le ventilateur médical délivre de l'air ou un mélange oxygène/azote (i.e. O₂/N₂).
- le ventilateur médical comprend une soufflante motorisée (i.e. aussi appelée turbine, compresseur ou analogue) délivrant le gaz respiratoire, typiquement de l'air ou un mélange oxygène/azote.
- le ventilateur médical comprend une soufflante motorisée commandée par des moyens de commande, telle une carte de commande électronique, agencés dans la carcasse du ventilateur médical.
- le ventilateur médical est de type HFO (High Frequency Oscillations), c'est-à-dire à oscillation à haute fréquence ou bien de type conventionnel, tel qu'un ventilateur de soins critiques.
- la source de NO contient un mélange gazeux NO/N₂ contenant entre 100 et 2000 ppmv de NO, le reste étant de l'azote (N₂), conditionné à une pression comprise entre 10 et 250 bar abs, typiquement à plus de 100 bar abs (avant début de soutirage).
- la source de NO contient un mélange gazeux NO/N₂ contenant entre 100 et 1000 ppmv de NO, le reste étant de l'azote (N₂), conditionné à une pression comprise entre 10 et 250 bar abs, typiquement à plus de 100 bar abs (avant début de soutirage).
- la source de NO est une (ou des) bouteille de gaz sous pression.
- la source de NO est une (ou des) bouteille de gaz ayant une contenance comprise entre 0,5 et 50 L (équivalent en eau).
- la bouteille de gaz comprend un corps cylindrique en acier ou en alliage d'aluminium.
- la bouteille de gaz est équipée d'un robinet simple (sans détendeur) ou à détendeur intégré ou RDI.
- la bouteille de gaz est équipée d'un RDI protégé par un capotage de protection, par exemple en métal ou polymère.
- le circuit patient comprend une branche inspiratoire et une branche expiratoire.
- le circuit patient comprend des conduites flexibles formant la branche inspiratoire et/ou la branche expiratoire, typiquement des tuyaux en polymère.
- la branche inspiratoire et la branche expiratoire, e.g. des conduites flexibles, sont raccordées à une pièce de jonction, telle une pièce en Y.
- la branche inspiratoire et/ou la branche expiratoire sont reliées fluidiquement à une interface respiratoire patient, de préférence via la pièce de jonction.
- l'interface respiratoire patient comprend une sonde d'intubation trachéale ou masque respiratoire.
- la branche inspiratoire et la branche expiratoire comprennent des conduites flexibles, par exemple en polymère.
- la branche inspiratoire et la branche expiratoire sont en outre fluidiquement raccordées à, respectivement, des orifices de sortie et d'entrée du ventilateur médical.
- la branche inspiratoire du circuit patient peut comprendre un humidificateur de gaz.
- l'humidificateur de gaz est agencé en aval du module d'injection de NO de manière à pouvoir humidifier le gaz avant son administration par inhalation au patient.
- la concentration ou posologie de NO dans le mélange gazeux final, c'est-à-dire le gaz qui est inhalé par le patient, après mélange du mélange NO/N₂ avec le gaz respiratoire contenant environ au moins 21%vol. d'oxygène provenant du ventilateur, tel de l'air ou un mélange O₂/N₂, est comprise entre 1 et 80 ppm en volume (ppmv), en fonction de la population traitée, i.e. nouveau-nés ou adultes, de l'état du patient et/ou de la maladie à traiter.

L'invention concerne aussi une utilisation d'une installation de fourniture de gaz et/ou du dispositif de délivrance de NO selon l'invention équipé du système de dosage de secours de NO de l'invention sont particulièrement bien adaptés à une utilisation dans le cadre d'une méthode de traitement thérapeutique avec fourniture d'un mélange gazeux comprenant de 1 à 80 ppmv de NO (posologie) et environ au moins 21%vol. d'oxygène et généralement de l'azote à un patient (i.e. une personne adulte, un enfant, un adolescent ou un nouveau-né), en ayant besoin, lequel patient souffre d'hypertensions pulmonaires et/ou d'hypoxie, engendrant des vasoconstrictions pulmonaires ou analogues, typiquement résultant ou causés par une ou des pathologies ou troubles pulmonaires de type PPHN (hypertension pulmonaire persistante du nouveau-né) ou SDRA (syndrome de détresse respiratoire aigüe), ou encore résultant ou engendrées par une opération de chirurgie cardiaque avec mise du patient sous circulation sanguine extracorporelle (CEC).

L'invention va maintenant être mieux comprise grâce à la description détaillée suivante, faite à titre illustratif mais non limitatif, en référence aux figures annexées parmi lesquelles :
- Fig. 1 schématise une installation de délivrance de gaz équipée d'un système de dosage d'urgence de NO selon la présente invention ;
- Fig. 2 schématise l'association entre orifice calibré et actionneur du système de dosage d'urgence de NO de Fig. 1 ; et
- Fig. 3 à Fig. 5 schématisent le fonctionnement de l'association orifice calibré/actionneur de Fig. 2.

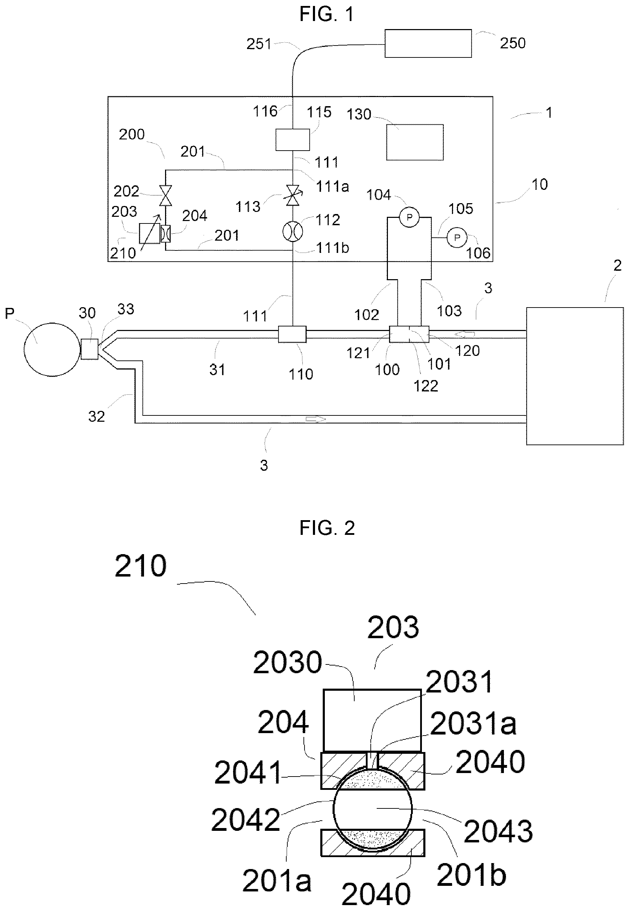

Fig. 1 schématise un mode de réalisation d'une installation de délivrance de gaz 1, 2 selon la présente invention comprenant un dispositif de délivrance de NO 1 comprenant un mécanisme de dosage d'urgence de NO, associé à un ventilateur mécanique 2, c'est-à-dire un appareil d'assistance respiratoire délivrant un gaz respiratoire, permettant de délivrer du NO sous forme gazeuse à un patient à une concentration désirée correspondant à une posologie fixée par un médecin-anesthésiste ou analogue, typiquement entre 1 et 80 ppmv de NO (i.e. ppm en volume).

Le ventilateur médical 2 délivre un gaz respiratoire contenant au moins 21% d'oxygène, tel que de l'air ou un mélange NO/N₂, dans un circuit patient 3, en particulier dans une branche inspiratoire 31 dudit circuit patient 3, servant à acheminer et à fournir le gaz respiratoire à un patient P pendant ses phases inspiratoires, c'est-à-dire de fournir une assistance respiratoire au patient P, et à convoyer les gaz expirés par le patient lors de ses phases expiratoires.

Le ventilateur médical 2 est un appareil classique comprenant par exemple une soufflante motorisée, aussi appelée turbine ou compresseur, délivrant le gaz respiratoire dans le circuit patient 3 et dont le fonctionnement est contrôlé par une (ou des) carte électronique de commande ou analogue. Il est alimenté électriquement par des moyens d'alimentation électrique, tel le secteur (110/220V) et/ou une batterie interne.

Comme visible sur Fig. 1, le circuit patient 3 comprend ici une branche inspiratoire 31 et une branche expiratoire 32 reliées fluidiquement à une pièce Y 33 ou similaire, en communication fluidique avec une interface respiratoire 30 permettant de délivrer le gaz au patient P ou bien de collecter les gaz expirés par ledit patient P. L'interface respiratoire 30 peut par exemple être un masque facial ou une sonde d'intubation.

Les branches inspiratoire 31 et expiratoire 32 comprennent des conduits, canalisations, tuyaux, passages, tubulures ou similaires, par exemple des tuyaux flexibles en polymère, aptes à et configurés pour convoyer les flux gazeux.

Le gaz respiratoire circulant dans la branche inspiratoire 31 du circuit patient 3, c'est-à-dire allant du ventilateur mécanique 2 au patient P, est inhalé par le patient P tandis que les gaz expirés par ledit patient P, i.e. des gaz enrichis en CO₂, empruntent la branche expiratoire 32 du circuit patient 3 en direction du ventilateur mécanique 2 pour être déchargés à l'atmosphère par le ventilateur mécanique 2.

Par ailleurs, un capteur de débit 100 ainsi qu'un module d'injection de NO 110 sont disposés dans la branche inspiratoire 31 du circuit patient 3. Le capteur de débit 100 est préférablement disposé dans la branche inspiratoire 31 entre le module d'injection de NO 110 et le ventilateur mécanique 2.

La branche inspiratoire 31 peut également comprendre un humidificateur (non représenté) afin d'humidifier le gaz délivré au patient P. Préférablement, l'humidificateur est placé en aval du module d'injection de NO 110, c'est-à-dire entre ledit module d'injection de NO 110 et l'interface respiratoire 30 fournissant le gaz au patient P.

Le capteur de débit 100 est utilisé afin de mesurer le flux gazeux, i.e. un débit, délivré par le ventilateur mécanique 2 et circulant dans la branche inspiratoire 31. Le capteur de débit 100 peut être un capteur du type mesurant et fournissant des signaux ou mesures de débit proprement-dits, par exemple un capteur de débit massique, ou du type mesurant et fournissant des signaux ou mesure de pression qui sont converties ensuite en débit par les moyens de pilotage, par exemple un capteur à mesure de pression différentielle, aussi appelé capteur de pression différentielle.

Dans le mode de réalisation de Fig. 1, le capteur de débit 100 est du type à mesure de pression différentielle, c'est-à-dire que le capteur de débit 100 comprend un module de mesure 100-1 comprenant une restriction interne 101 qui crée une perte de charge engendrant un différentiel ou gradient de pression lorsqu'un débit gazeux traverse cette restriction interne 101.

Comme on le voit en Fig. 1, le module de mesure 100-1 du capteur de débit 100 comprend des chambres amont 120 et aval 121 qui sont séparées par une paroi 122 traversées un passage de gaz de sorte de former la restriction interne 101. Une ligne amont 103 et une ligne aval 102 de mesure de pression, i.e. des lignes pneumatiques, telles des conduits de gaz, sont connectées fluidiquement au module de mesure 100-1 du capteur de débit 100 en des sites de raccordement situés en amont et en aval de la restriction interne 101, en particulier aux chambres amont 120 et aval 121 afin d'y opérer les mesures de pression du flux circulant, avant et après perte de charge.

La différence de pression créée par la restriction interne 101 est déterminée par un capteur de pression différentiel 104 raccordé au capteur de débit 100 par le biais des lignes amont 102 et aval 103 qui forment des conduits de mesure de pression et fournissent au capteur de pression différentiel 104, les mesures de pression du flux circulant, avant et après perte de charge. Préférentiellement, le capteur de pression différentiel 104 est intégré dans le boitier 10 du dispositif de délivrance de NO 1. Le capteur 104 peut être soit connecté électriquement à une unité de pilotage 130, aussi appelée moyens de pilotage 130, soit peut lui transmettre les mesures de pression afin qu'elles y soient traitées informatiquement.

L'unité de pilotage 130, i.e. les moyens de pilotage 130, comprend un système de traitement de données, i.e. de mesures, comprenant par exemple un (ou des) microprocesseur(s) agencé sur une (ou des) carte électronique et mettant en œuvre un (ou des) algorithme(s), i.e. un (ou des) programme d'ordinateur.

En d'autres termes, l'unité de pilotage 130 est configurée pour traiter et/ou exploiter les signaux de mesure de pression ou les valeurs de pression transmises par le capteur de pression différentielle 104 coopérant avec le capteur de débit 100.

Par exemple, l'unité de pilotage 130 dispose d'une table de correspondance préenregistrée qui permet la détermination du débit de gaz circulant dans la branche inspiratoire 31 et le module de mesure 100-1 du capteur de débit 100, c'est-à-dire de transformer une valeur de pression transmise par le capteur de pression différentiel 104 en valeur de débit traversant le module de mesure 100-1 du capteur de débit 100.

Une telle détermination du débit traversant le capteur de débit 100 permet ensuite de calculer la quantité de NO devant être ajoutée au gaz circulant dans la branche inspiratoire 31 avant d'atteindre le patient P.

En d'autres termes, en utilisant la mesure de pression retournée par le capteur de pression différentielle 104 et la table de correspondance, l'unité de pilotage 130 peut déterminer le débit de gaz issu du ventilateur mécanique 2 et la quantité de NO devant être ajoutée, via le module d'injection de NO 110, afin d'obtenir la concentration de NO souhaitée, i.e. la posologie définie par le médecin, devant être inhalée par le patient P.

Bien entendu, l'unité de pilotage 130 peut être aussi configurée pour piloter d'autres éléments électromécaniques intégrés dans le boitier 10 ou carcasse externe du dispositif de délivrance de NO 1.

Selon un autre mode de réalisation (non montré), le capteur de débit 100 pourrait être aussi un capteur de débit massique ou analogue qui serait relié directement aux moyens de pilotage 130, via une (ou des) liaisons électriques, tels un ou des câbles ou analogues, pour leur fournir un signal, telle une tension, ou une mesure du débit traversant le capteur 100. Dans ce cas, le capteur de pression différentielle 104 est supprimé.

D'une façon générale, le mélange gazeux final, i.e le gaz respiratoire à base de NO qui est ensuite administré par inhalation au patient, obtenu au niveau du module d'injection de NO 110 agencé sur la branche inspiratoire 31 comprend alors principalement de l'azote (N₂), de l'oxygène (O₂), typiquement en une teneur d'environ au moins 21%vol., et du NO à une teneur typiquement comprise entre 1 et 80 ppmv, par exemple de l'ordre de 10 à 20 ppmv.

Plus précisément, pendant un fonctionnement normal, en fonction du débit gazeux (i.e. air ou N₂/O₂) circulant dans la branche inspiratoire 31 ayant été déterminé à l'aide du capteur de débit 100, l'unité de pilotage 130 détermine la quantité de NO, typiquement un mélange de NO et de N₂, devant être ajouté au gaz circulant dans la branche inspiratoire 31 par le module d'injection de NO 110, afin d'obtenir la concentration ou posologie souhaitée, c'est-à-dire fixée par le médecin ou analogue, pendant le fonctionnement normal de l'installation de fourniture de gaz 1, 2.

Typiquement, la concentration ou posologie de NO dans le gaz inhalé par le patient, après mélange du mélange NO/N₂ avec l'air ou un mélange O₂/N₂, est comprise entre 1 et 80 ppm en volume (ppmv), par exemple de l'ordre de 10 à 20 ppmv, en fonction de la population traitée, i.e. nouveau-nés ou adultes, et donc de la maladie à traiter.

Le dispositif de délivrance de NO 1 est alimenté en NO gazeux, typiquement en mélange NO/N₂ gazeux, provenant d'une source de NO 250 reliée fluidiquement au dispositif de délivrance de NO 1, en particulier à une ligne haute pression 116 dudit dispositif de délivrance de NO 1, par une ligne d'alimentation 251, telle un conduit flexible ou analogue.

Typiquement, la source de NO 250 est une (ou des) bouteille de gaz sous pression renfermant un mélange de NO/N₂ contenant une concentration en NO généralement comprise entre 100 et 2000 ppmv, de préférence entre 200 et 1000 ppmv, par exemple de l'ordre de 800 ppmv, et conditionné à une pression (lorsque totalement pleine) pouvant atteindre 200 à 250 bar abs, voire plus.

Le mélange NO/N₂ est fourni au module d'injection 110 par le dispositif de délivrance de NO 1 via une ligne d'injection 111, telle une conduite de gaz flexible.

La ligne d'injection 111 située dans le boitier 10 du dispositif de délivrance de NO 1 est fluidiquement connectée à une ligne haute pression 116 du dispositif de délivrance de NO 1, laquelle ligne haute pression 116 présente une entrée haute pression connectée fluidiquement à la source de NO pour être alimentée en NO/N₂ sous pression, c'est-à-dire à une pression pouvant atteindre 200 bar abs.

La ligne haute pression 116, par exemple un passage ou conduit de gaz, est aussi agencée dans le boitier 10 du dispositif de délivrance de NO 1 et comprend un régulateur de pression 115 qui réduit la pression du mélange NO/N₂ à une valeur stable, par exemple 4 bar abs. Le port de sortie du régulateur de pression 115 fourni une pression stable dans la portion amont de la ligne d'injection 111.

Un dispositif à vanne 113, telle une électrovanne, de préférence une électrovanne proportionnelle, telle que la série VSO miniature de Parker par exemple, est agencé dans le dispositif 1 afin de contrôler le débit de NO gazeux au sein de la ligne d'injection 111. Le débit gazeux circulant dans la ligne d'injection 111 est mesuré par un dispositif de mesure de débit 112, aussi appelé capteur de débit de NO, agencé sur la ligne d'injection 111, préférentiellement placé en aval de l'électrovanne 113, comme visible sur Fig. 1.

Le régulateur de pression 115, le dispositif à vanne ou électrovanne 113, le capteur de débit de NO 112 et une portion de la ligne d'injection 111 sont agencés dans le boitier 10 du dispositif de délivrance de NO 1.

Le dispositif à vanne 113 est configuré pour être normalement dans une position fermée (i.e. un état de repos) pour empêcher toute circulation de gaz dans la ligne d'injection 111.

Avantageusement, dans l'état de repos, le dispositif à vanne 113 n'est pas piloté par les moyens de pilotage 130. Autrement dit, lorsque les moyens de pilotage 130 stoppent/cessent de commander le dispositif à vanne 113, il (re)passe automatiquement en état de repos, c'est-à-dire en position fermée.

A l'inverse, pour passer en position ouverte, le dispositif à vanne 113 doit être commandé par les moyens de pilotage 130, comme c'est le cas pendant le fonctionnement normal de l'installation de fourniture de gaz 1, 2.

Par ailleurs, selon l'invention, on prévoit un système de dosage de secours 200 de NO agencé dans le boitier 10 du dispositif de délivrance de NO 1 et utilisé en cas de défaillance, comme détaillé ci-après.

Le système de dosage de secours 200 de NO comprend une ligne de secours 201, aussi appelée ligne de bipasse, tel un passage de gaz, un conduit de gaz ou analogue. La ligne de secours 201 vient se connecter fluidiquement à la ligne d'injection 111 en un premier site de raccordement 111a situé en amont du dispositif à vanne 113, telle une électrovanne proportionnelle, et ici en aval du régulateur de pression 115, et en un second site de raccordement 111b situé en aval du dispositif à vanne 113 et, de préférence, en aval du capteur de débit de NO 112.

Autrement dit, un dispositif à vanne 113, telle une électrovanne proportionnelle, et préférentiellement le capteur de débit de NO 112 sont situés entre les premier et second sites de raccordement 111a, 111b de la ligne de secours 201, c'est-à-dire que la ligne de secours 201 bipasse, le dispositif à vanne 113 et préférentiellement le capteur de débit de NO 112 situés sur la ligne d'injection 111. Selon un autre mode de réalisation, le second site de raccordement 111b pourrait être situé en aval du dispositif à vanne 113 et en amont du capteur de débit de NO 112, c'est-à-dire entre ces deux éléments.

La ligne de secours 201 comprend quant à elle une électrovanne de secours 202 et un dispositif de contrôle de débit 210 faisant partie du système de dosage de secours 200 de l'invention. Cette électrovanne de secours 202 est configurée pour être normalement dans une position ouverte (i.e. état ouvert) pour permettre une circulation de gaz dans la ligne de secours 201.

Le dispositif de contrôle de débit 210 forme en fait un système à orifice calibré 204 de type proportionnel permettant d'ajuster ou régler le débit de gaz de secours, i.e. mélange NO/N₂, circulant dans la ligne de secours 201. Il peut revêtir différentes formes, i.e. agencements, notamment celui illustré sur Fig. 2 à Fig. 5 et détaillé ci-après.

L'électrovanne de secours 202 est préférentiellement une électrovanne de type « tout ou rien » ayant deux états possibles, à savoir un état ouvert et un état fermé, par exemple une électrovanne de la série Picosol de IMI Norgren. L'électrovanne de secours 202 est normalement ouverte, c'est-à-dire qu'en l'absence d'une commande électrique venant de l'unité de pilotage 130, l'électrovanne de secours 202 est dans son état de repos, c'est-à-dire en position ouverte, ce qui permet alors au gaz issu de la source de NO d'emprunter la ligne de secours 201 depuis le premier site de raccordement 111a en direction du second site de raccordement 111b.

Autrement dit, comme précédemment pour le dispositif à vanne 113, dans son état de repos, l'électrovanne de secours 202 n'est pas pilotée par les moyens de pilotage 130. Autrement dit, lorsque les moyens de pilotage 130 stoppent/cessent de commander l'électrovanne de secours 202, elle (re)passe aussi automatiquement en position ouverte qui correspond à son état de repos.

Là encore, ce sont les moyens de pilotage ou unité de pilotage 130, qui assurent la fermeture de l'électrovanne de secours 202, c'est-à-dire son passage de l'état de repos (i.e. position ouverte) à son état actif, c'est-à-dire en position fermée, comme c'est le cas pendant le fonctionnement normal de l'installation de fourniture de gaz 1, 2.

Autrement dit, les moyens de pilotage 130, i.e. l'unité de pilotage, sont configurés pour coopérer avec l'électrovanne de secours 202, le dispositif de contrôle de débit 210, le dispositif à vanne 113 et le dispositif de mesure de débit 112, lors d'un fonctionnement normal du dispositif 1 et de l'installation de fourniture de gaz 1, 2.

Dans le mode de réalisation présenté sur Fig. 2 à Fig. 5, le dispositif de contrôle de débit 210 comprenant ou formant un système à orifice calibré proportionnel, c'est-à-dire constituant, incluant ou formant un orifice calibré 204 de type proportionnel, comprend un moyen actionneur 203 coopérant avec un élément mobile 2042 agencé dans un compartiment 2041, ledit élément mobile 2042 comprenant un évidement traversant 2043.

Toutefois, le dispositif de contrôle de débit 210 constituant l'orifice calibré 204 de type proportionnel peut revêtir une autre forme, par exemple un dispositif de type à vanne à pointeau ou encore un ensemble comprenant un régulateur de pression agencé en amont d'un orifice fixe, ledit régulateur de pression pouvant être réglé à différentes pressions de sortie, générant ainsi différents débits traversant ledit orifice fixe.

Ainsi, Fig. 2 est un schéma en coupe d'un mode de réalisation du dispositif de contrôle de débit 210, i.e. de l'orifice calibré 204, qui est formé ici par le moyen actionneur 203 et l'élément mobile 2041 associé à l'évidement traversant 2043, du système de dosage d'urgence de NO équipant le dispositif de délivrance 1 de NO de l'invention.

Le moyen actionneur 203, plus simplement appelé actionneur, de Fig. 2 est préférentiellement un moteur de type pas à pas 2030, par exemple comme celui commercialisé par la société Portescap, prolongé par un axe 2031 mécaniquement couplé en 2031a à l'élément mobile 2042.

Par ailleurs, l'élément mobile 2042 est ici une sphère. La sphère formant l'élément mobile 2042 peut être métallique, par exemple en acier inoxydable et présente un évidement traversant 2043, c'est-à-dire qu'elle est diamétralement traversée par un perçage ou passage interne permettant le passage du gaz. L'élément mobile 2042, i.e. la sphère, est logé dans un compartiment ou logement interne 2041 formant une chambre à sphère qui est ici de forme générale sphérique. Le logement 2041 est aménagé dans une pièce formant corps 2040. Le diamètre externe de la sphère 2042 est sensiblement égal au diamètre interne du logement interne 2041.

La pièce formant corps 2040 peut être aussi métallique, par exemple une bille d'acier ou analogue. Elle comprend un port d'entrée 201a et un port de sortie 201b en communication fluidique avec le logement interne 2041.

Sur Fig. 2, on voit que l'évidement traversant 2043 de la sphère 2042 est aligné avec les ports d'entrée 201a et de sortie 201b du corps 2040, qui sont en communication fluidique avec la ligne de secours 201, c'est-à-dire qu'ils sont en continuité fluidique, de sorte que le gaz peut s'écouler du port d'entrée 201a vers le port de sortie 201b via l'évidement traversant 2043 de la sphère 2042.

Comme indiqué, le moyen actionneur 203 est ici un moteur pas à pas 2030 entraînant en rotation l'axe 2031 et donc la sphère 2042. En réponse à une commande provenant de l'unité de pilotage 130, le moteur 2030 pas à pas va adopter une position différente et faire subir une rotation à l'axe 2031 qui va alors entraîner la sphère 2042 aussi en rotation.

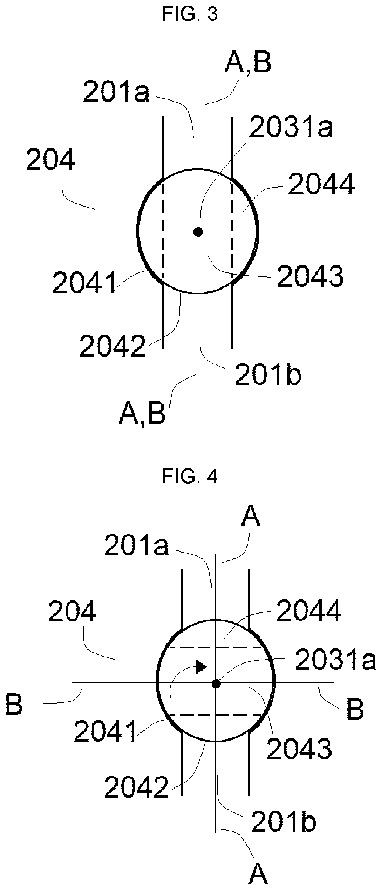

En considérant que l'unité de pilotage 130 est en capacité de faire varier la valeur de commande de façon proportionnelle, il s'ensuit que l'axe 2031 peut subir des mouvements de rotation plus ou moins importants et de façon proportionnelle, par exemple entre 0 et 90°. Autrement dit, le mouvement de rotation subi par l'axe 2031 est donc transmis à la sphère 2042 qui pivote en réponse, au sein de son logement 2041, comme illustré sur Fig. 3 à Fig. 5, ce qui permet de régler ou ajuster le débit de gaz souhaité, pendant le fonctionnement normal de l'appareil 1.

Ainsi, Fig. 3 est un schéma de dessus de l'orifice calibré 204 de Fig. 2 montrant, comme déjà expliqué, l'axe 2031 qui impose à la sphère 2042 de présenter son évidement 2043 en continuité des ports d'entrée 201a et de sortie 201b du corps 2040 de sorte à créer une connexion fluidique entre lesdits ports d'entrée 201a, 201b et autoriser ainsi le passage de gaz au travers des ports 201a, 201b et de l'évidement 2043. L'orifice calibré 204 est alors à son ouverture maximale, c'est-à-dire en position totalement ouverte. Dans cette position, l'axe AA de l'évidement traversant 2043 de la sphère 2042 est (quasi)coaxial à l'axe BB passant par les ports d'entrée (201a) et de sortie (201b) de sorte que l'ouverture soit maximale, donc le flux maximal dans la ligne de secours 201, y compris au travers de l'évidement traversant 2043 de la sphère 2042.

En Fig. 4, l'unité de pilotage 130 a commandé le moteur pas à pas 2030 pour faire subir à l'axe 2031, une rotation ici de l'ordre de 90° et par conséquent aussi à la sphère 2042 qui subit aussi la même rotation de 90°. Après rotation, les ports d'entrée 201a et de sortie 201b du corps 2040 de l'orifice calibré 204 ne font plus face à l'évidement 2043 de la sphère 2042 mais à une portion 2044 non-évidée, i.e. pleine, de la sphère 2042 et se retrouvent alors totalement obturés par la partie non-évidée 2044 de la sphère 2042. La connexion fluidique est alors rompue et aucun gaz ne peut circuler entre les ports d'entrée 201a et de sortie 201b du corps 2040 de l'orifice calibré 204. L'orifice calibré 204 est alors totalement fermé.

Dans cette position dite fermée, l'axe AA de l'évidement traversant 2043 de la sphère 2042 est (quasi)perpendiculaire à l'axe BB passant par les ports d'entrée (201a) et de sortie (201b) de sorte qu'aucun passage de gaz n'a lieu au travers de l'évidement traversant 2043, donc dans la ligne de secours 201.

Entre Fig. 3 et Fig. 4, l'unité de pilotage 130 a imposé à l'actionneur 203 des valeurs de commandes extrêmes, i.e. entre 0° et 90° de rotation, permettant d'obtenir soit une communication complète (cf. Fig. 3), soit une isolation complète (cf. Fig. 4) des ports d'entrée 201a et de sortie 201b du corps 2040 de l'orifice calibré 204.

Toutefois, l'unité de pilotage 130 est aussi configurée pour pouvoir assigner, de façon proportionnelle, des commandes entrainant une rotation de l'axe 2031 et de la sphère 2042 entre ces deux positions angulaires extrêmes, i.e. 0° et 90° de rotation, c'est-à-dire un angle non nul mais inférieur à 90°.

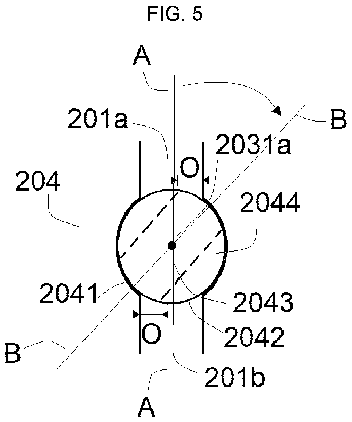

Ainsi, Fig. 5 donne l'exemple une position angulaire intermédiaire où la sphère 2042 a subi un mouvement de rotation de l'ordre de 45°. Dans ce cas, le port d'entrée 201a du corps 2040 de l'orifice calibré 204 est partiellement obstrué, c'est-à-dire exposé à des parties non-évidée 2044 et évidée 2043 de la sphère 2042. La section de passage de gaz de la partie évidée 2043 de la sphère 2042 en relation fluidique avec le port d'entrée 201a est alors définie par un niveau (ou taille) d'ouverture O. Cette section de passage de gaz est toujours inférieure à la section maximale de connexion fluidique telle que représentée en Fig. 3. Par le biais de la rotation axiale, le même niveau d'ouverture O apparaît entre le port de sortie 201b et la partie évidée 2043 de la sphère 2042 du corps 2040 de l'orifice calibré 204.

Dans les positions dites intermédiaires, l'axe AA de l'évidement traversant 2043 de la sphère 2042 et l'axe BB passant par les ports d'entrée (201a) et de sortie (201b) forment entre eux un angle variable compris strictement ici entre 0 et 90° de sorte que le passage de gaz au travers de l'évidement traversant 2043, donc dans la ligne de secours 201, soit limité/réduit mais non-nul, ni maximum, c'est-à-dire en fonction de l'ouverture O désirée de l'orifice calibré 204.

Ainsi, en fonction de la commande imposée sur l'actionneur 203 par l'unité de pilotage 130, le niveau d'ouverture O défini par l'intersection des ports d'entrée 201a, de sortie 201b et de la partie évidée 2043 de la sphère 2042, varie d'une valeur nulle (Fig. 3) à une valeur maximale (Fig. 4), c'est-à-dire peut prendre les valeurs intermédiaires situées entre ces deux valeurs extrêmes, i.e. entre 0 et 90°, ce qui permet de régler ou ajuster le débit de gaz circulant dans la ligne de secours 201.

En effet, on comprend aisément que chaque niveau ou valeur d'ouverture O correspond à un orifice calibré équivalent dont le diamètre de passage du gaz est fonction du positionnement de la sphère 2042 et par conséquent de la commande envoyée par l'unité de pilotage 130, i.e. les moyens de pilotage, à l'actionneur 203.

Comme déjà dit, cet ensemble forme donc bien un orifice calibré proportionnel puisque son calibre ou niveau d'ouverture O varie en fonction de la position angulaire prise par la sphère 2042 au sein du corps 2040 de l'orifice calibré 204.

Or, la pression régnant dans la portion amont de la ligne de secours 201, c'est-à-dire en amont de l'orifice calibré 204, est stable et connue puisqu'elle correspond à la pression de détente du régulateur de pression 115 fixée par exemple à 4 bar abs. Le débit de gaz circulant dans la portion aval de la ligne de secours 201, c'est-à-dire en aval de l'orifice calibré 204, est donc fonction du niveau d'ouverture O.

Ainsi, l'unité de pilotage 130 peut disposer d'une table de correspondance reliant un niveau de commande donné à un niveau d'ouverture et à un débit de gaz traversant l'orifice calibré 201 en direction de la ligne d'injection 111 et y pénétrant au second site de raccordement 111b.

Pour des raisons de simplification, on admet que le niveau de pression régnant dans la branche inspiratoire 31 du circuit patient 3, et donc dans le module d'injection de NO 110 et la ligne d'injection 111 est négligeable au regard de la pression de détente du régulateur de pression 115 et n'a donc pas d'impact sur la précision des mesures de débit circulant dans la ligne de secours 201 réalisées par l'unité de pilotage 130.

Bien entendu, selon un mode de réalisation particulier, des moyens de mesure supplémentaires, tel qu'un dispositif de mesure de pression additionnel agencé pour opérer une mesure de pression en aval de l'orifice calibré 204 de la ligne de secours 201 peuvent être implémentés, i.e. utilisés, à des fins de compensation, sans changer de quelle que manière que ce soit l'objet de la présente invention.

Enfin, il est à noter que le choix d'un moteur pas à pas est particulièrement recommandé car, contrairement aux électrovannes 202, 113 qui prendront une position de repos, en cas de coupure d'alimentation, à savoir une position ouverte pour l'électrovanne tout ou rien 202 et une position fermée pour l'électrovanne proportionnelle 113, la position du moteur pas à pas reste permanente et fixée selon la dernière commande imposée. Autrement dit, l'orifice calibré 204 présente un niveau d'ouverture fixe correspondant à la dernière valeur de commande reçue par le moteur pas à pas, i.e. la dernière commande provenant des moyens de pilotage 130.

Bien entendu, la présente invention ne se limite pas à un actionneur de type moteur pas à pas. En effet, tout autre actionneur gardant sa position en cas de défaut d'alimentation électrique et pouvant être couplé à un mécanisme mécanique permettant de définir un orifice calibré à taille variable peut être utilisé, comme par exemple un moteur linéaire ou autre.

Par ailleurs, le dispositif de délivrance de NO 1 est alimenté électriquement par une alimentation électrique, tel le secteur (110/220V) ou une batterie interne, afin de permettre le bon fonctionnement de ses composants nécessitant du courant électrique pour fonctionner, notamment l'actionneur 203, tel un moteur électrique pas à pas, l'unité de pilotage 130, les électrovannes 202, 113, ou autres.

En outre, le dispositif de délivrance de NO 1 comprend aussi des moyens de mémorisation, telle une mémoire informatique, pour mémoriser des données, informations ou autres, par exemple une ou des tables de correspondances, les mesures de débit de gaz opérées par le dispositif de mesure de débit 112 ou provenant du capteur de débit 100 et/ou traitées par les moyens de pilotage 130, ou autres.

Selon l'invention, en cas de défaillance du fonctionnement de l'installation 1, 2 résultant d'une perte du signal, i.e. des mesures, provenant du capteur de débit 100, par exemple par en cas de déconnexion des lignes amont 103 et/ou ligne aval 102 de mesure de pression, lesquelles peuvent être couplées mécaniquement, on doit pouvoir continuer à assurer un traitement du patient avec du NO inhalé et ce, malgré le dysfonctionnement.

Pour ce faire, en cas de perte de signal par les moyens de pilotage 130 du (des) signal provenant du capteur de débit 100, l'électrovanne de secours 202 cesse d'être commandée par les moyens de pilotage 130, comme elle l'est pendant le fonctionnement normal de l'installation 1, 2, de manière à passer automatiquement en position ouverte, c'est-à-dire dans son état de repos, pour permettre une circulation d'un flux de gaz de secours (i.e. mélange NO/N₂) dans la ligne de secours 201 et, dans le même temps, le dispositif à vanne 113 cesse lui aussi d'être commandé par les moyens de pilotage 130, pour passer en position fermée, c'est-à-dire dans son état de repos, afin de stopper toute circulation de gaz (i.e. mélange NO/N₂) dans la ligne d'injection 111, ce qui permet de fournir le gaz (i.e. mélange NO/N₂) à un débit gazeux de secours, via la ligne de secours 201 et le dispositif de contrôle de débit 210, même en cas de perte de signal.

Dans ce cas, le débit gazeux de secours correspond à un débit de gaz préréglé au niveau du dispositif de contrôle de débit 210 formant un système à orifice calibré proportionnel, pendant le fonctionnement normal de l'installation, c'est-à-dire avant la perte de signal du capteur de débit 100.

Plus précisément, le débit gazeux de secours correspond au « dernier » débit ayant été calculé par les moyens de pilotage 130 à partir du ou des derniers signaux de mesure correspondant au(x) débit(s) de gaz respiratoire (e.g. air ou N₂/O₂) dans la branche inspiratoire, ayant été fourni par le capteur de débit 100 du circuit patient 3 aux moyens de pilotage 130, avant ladite interruption de réception dudit signal, et par ailleurs de la posologie en NO désirée, laquelle est typiquement comprise entre 1 et 80 ppmv. La posologie ou concentration en NO souhaitée dans le mélange final est fixée par le personnel soignant, e.g. un médecin ou analogue, et peut être mémorisée dans le dispositif de délivrance 1 de NO.

Préférentiellement, le débit gazeux de secours est calculé à partir de plusieurs valeurs de débit ayant été mesurées par le capteur de débit 100 au sein de la branche inspiratoire du circuit patient 3, pendant le fonctionnement normal de l'installation 1, 2. Lesdites valeurs de débit sont moyennées par les moyens de pilotage , c'est-à-dire que les moyens de pilotage 130 calculent un débit de gaz moyen ou « débit moyen » sur une période de temps donnée, durant laquelle les valeurs ont été opérées, par exemple pendant plusieurs secondes ou dizaines de secondes. Le débit gazeux de secours est ensuite préréglé au niveau du dispositif de contrôle de débit 210 formant le système à orifice calibré proportionnel, également pendant le fonctionnement normal de l'installation 1, 2.

Dit autrement, pendant le fonctionnement normal de l'installation 1,2 précédant le dysfonctionnement, le débit gazeux de secours est déterminé par les moyens de pilotage 130 à partir de la ou des dernières mesures de débit du gaz fournies par le capteur de débit 100, en particulier un débit moyen, comme expliqué ci-avant, et éventuellement mémorisée(s), et bien entendu, de la posologie désirée, i.e. teneur en NO dans le mélange final. Les moyens de pilotage 130 peuvent alors agir immédiatement, c'est-à-dire avant toute perte de signal éventuelle future, sur le dispositif de contrôle de débit 210 du dispositif de fourniture de NO 1 pour ajuter l'orifice calibré qui s'y trouve pour qu'il soit apte à délivrer immédiatement un flux de gaz de secours (i.e. mélange NO/N₂) au débit gazeux de secours désiré via la ligne de secours 201, dès qu'une perte de signal est détectée et que les moyens de pilotage 130 cessent ou stoppent leur commande de l'électrovanne de secours 202 et du dispositif à vanne 113 qui passent alors dans leurs états de repos respectifs, à savoir en position ouverte pour l'électrovanne de secours 202 et en position fermée pour le dispositif à vanne 113 de manière à assurer une circulation du mélange gazeux NO/N₂ dans la ligne de secours 201 mais à empêcher ou stopper toute circulation de gaz dans la ligne d'injection 111.

Les moyens de pilotage 130 déterminent donc le débit gazeux de secours, avant toute perte de signal, à partir de la posologie de NO désirée et de la ou des dernières mesures de débit du gaz ayant été fournies par le dispositif de mesure de débit 100, pendant le fonctionnement normal, c'est-à-dire avant interruption de signal, et agissent sur le dispositif de contrôle de débit 210 pour y prérégler ce débit gazeux de secours, par exemple en jouant sur le calibre ou niveau d'ouverture O agissant sur la position angulaire prise par la sphère 2042 au sein du corps 2040 de l'orifice calibré 204, comme expliqué ci-avant. De préférence, les dernières mesures de débit sont utilisées par les moyens de pilotage 130 pour calculer un débit moyen sur une durée donnée de quelques secondes ou dizaines de secondes précédant le dysfonctionnement et c'est ce débit moyen qui est utilisé pour déterminer le débit gazeux de secours permettant d'obtenir la posologie désirée.

Plus précisément, le fonctionnement du système de dosage de secours 200 de NO du dispositif de délivrance de NO 1 de l'invention est globalement le suivant.

Comme illustré en Fig. 1, le dispositif de délivrance de NO 1 coopère avec un ventilateur mécanique 2 afin d'apporter une aide thérapeutique au patient P. Comme déjà expliqué, le débit du flux de gaz respiratoire contenant de l'oxygène, typiquement environ au moins 21%vol. d'O₂, (e.g. air ou N₂/O₂, voire de l'O₂ pur) issu du ventilateur mécanique 2 et circulant dans la branche inspiratoire 31 du circuit patient 3 est mesuré en permanence par le capteur de débit 100 et transmis à l'unité de pilotage 130, comme détaillé ci-avant selon que le capteur de débit 100 soit un capteur de débit proprement-dit ou à mesure de pression différentielle.

Ces mesures de débit permettent à l'unité de pilotage 130 de déterminer, en temps réel, le débit de NO, i.e. débit de secours, devant être injecté dans la ligne d'injection 111 et le module d'injection de NO 110 afin de satisfaire la concentration ou posologie de NO souhaitée dans le gaz fourni au patient, à savoir entre 1 et 80 ppmv, typiquement entre 5 et 80 ppmv, par exemple de l'ordre de 10 à 20 ppmv.

En fonctionnement normal, l'unité de pilotage 130 commande le dispositif à vanne 113, de préférence une électrovanne proportionnelle, en position ouverte (i.e. état actif) pour permettre une circulation du mélange NO/azote dans la ligne d'injection 111. A l'inverse, afin de ne pas introduire de débit additionnel provenant de la ligne de secours 201 dans la ligne d'injection 111, l'unité de pilotage 130 commande l'électrovanne 202, qui est préférentiellement de type tout ou rien, en position fermée (i.e. état actif) et, en parallèle, va piloter l'actionneur 203, i.e. moteur pas à pas, afin de prérégler l'orifice calibré 204 en définissant un niveau d'ouverture O donné, donc un débit de secours.

Ceci est opéré de la façon suivante par l'unité de pilotage 130, à partir d'une ou de préférence plusieurs mesures de débit de gaz respiratoire (e.g. air ou N₂/O₂, voire O₂) provenant du ventilateur 2, lequel circule dans la branche inspiratoire 31 du circuit patient 3 à un (des) débit déterminé par le capteur de débit 100, qui est transmis aux moyens de pilotage 130.

L'unité de pilotage 130 utilisent les mesures de débit de gaz respiratoire pour préférentiellement calculer un débit moyen sur une durée donnée, par exemple de quelques secondes, et ensuite évaluer la valeur de débit de NO (en L/min) permettant d'obtenir ou de se rapprocher de la concentration en NO ou posologie souhaitée, e.g. entre 1 et 80 ppmv, par exemple de l'ordre de 10 à 20 ppmv, après mélange du flux de mélange NO/azote amené par la ligne d'injection 111 avec le gaz respiratoire circulant dans la branche inspiratoire 31 du circuit patient 3. Autrement dit, le calcul de la valeur de débit de NO prend en compte, pendant le fonctionnement normal de l'installation, le débit de gaz respiratoire mesuré dans le circuit patient 3 par le capteur de débit 100, en particulier un débit moyen calculé par les moyens de pilotage 130.

Plus précisément, lorsque la valeur de débit de secours a été déterminée, l'unité de pilotage 130 réalise une conversion par le biais d'une table de correspondance mémorisée de manière à commander l'actionneur 203 du dispositif de contrôle de débit 210 et par conséquent définir un niveau d'ouverture de l'orifice calibré 204 afin de fixer un débit de NO pouvant circuler dans la ligne de secours 201 égal à la valeur calculée de NO moyen fixe, c'est-à-dire correspondant au débit de secours préfixé. Il est à souligner que cette activité n'a pas d'effet physique, i.e. aucun débit de gaz ne circule dans la ligne de secours 201 car l'électrovanne tout ou rien 202 est fermée, pendant le fonctionnement normal de l'installation 1, 2, c'est-à-dire avant toute perte de signal issu du capteur de débit 100.

Par contre, en cas d'interruption de signal provenant du capteur de débit 100, le dispositif à vanne 113, typiquement une électrovanne proportionnelle, retrouve son état de repos, à savoir passe dans sa position fermée empêchant tout passage de gaz, alors que l'électrovanne de secours 202 se retrouve simultanément dans son état de repos, à savoir sa position ouverte, autorisant le passage du gaz provenant de la source de NO dans la ligne de secours 201 et sa circulation jusqu'à atteindre le second site de jonction 111b, puis la partie aval de la ligne d'injection 111. L'unité de pilotage 130 a alors cessé de piloter le dispositif à vanne 113 et l'électrovanne de secours 202 qui sont alors repassés automatiquement dans leur état de repos.

Dit autrement, les passages du dispositif à vanne 113, typiquement une électrovanne proportionnelle, et/ou de l'électrovanne 202 dans leur état de repos se font naturellement, c'est-à-dire indépendamment de toute commande par les moyens de pilotage 130. Ces états de repos sont en fait des états « par défaut » du dispositif à vanne 113 et de l'électrovanne 202.

Le mélange NO/N₂ peut alors circuler dans la ligne de secours 201 à un débit de secours calculé comme expliqué ci-avant et détaillé ci-après, qui est contrôlé par l'orifice calibré du dispositif de contrôle de débit 210. Le flux de NO/N₂ au débit de secours rejoint la ligne d'injection 111 (en 111b) et peut ensuite être injecté dans la branche inspiratoire 31 du circuit patient 3 via le module d'injection de NO 110, comme déjà expliqué, pour obtenir le mélange final à administrer au patient qui est à la posologie désirée en NO, c'est-à-dire habituellement entre 1 et 80 ppm de NO, par exemple de l'ordre de 10 à 20 ppmv.

On comprend donc que, selon l'invention, le dispositif de contrôle de débit 210 est configuré, i.e. préréglé pendant le fonctionnement normal de l'installation 1, 2, pour fournir, en cas de dysfonctionnement avec perte de signal du capteur de débit 100, le gaz, i.e. NO/N₂, à un débit gazeux de secours qui a été déterminé par les moyens de pilotage 130 à partir d'une (ou plusieurs) mesure de débit du gaz fournie par le capteur de débit 100, par exemple via les lignes amont et aval 103, 102, et le capteur de pression différentielle 104, avant dysfonctionnement, préférentiellement la dernière valeur de débit du gaz respiratoire provenant du ventilateur 2 et ayant été mesurée avant le dysfonctionnement, causé par exemple par une déconnexion intempestive ou accidentelle des lignes amont et aval 103, 102, voire une défaillance subite du capteur de débit 100.

La valeur de débit de NO est donc préréglée au sein du dispositif de contrôle de débit 210 par les moyens de pilotage 130, par exemple en agissant sur la position angulaire prise par la sphère 2042 au sein du corps 2040 de l'orifice calibré 204 du dispositif de contrôle de débit 210 afin d'y faire varier le calibre ou niveau d'ouverture O, comme expliqué ci-avant, par commande dudit dispositif de contrôle de débit 210 par les moyens de pilotage 130, ledit préréglage ayant lieu, c'est-à-dire étant opéré ou réalisé, pendant ledit fonctionnement normal du dispositif 1.

Bien entendu, le fait d'injecter un débit continu de NO dans la branche inspiratoire 31 du circuit patient 3 ne garantit pas une même précision de concentration en NO inhalé que lorsque le système de délivrance de NO 1 opère en fonctionnement normal, c'est-à-dire en ajustant le débit de NO en fonction du débit traversant le capteur de débit 100, mais le volume tampon généré par la portion de la branche inspiratoire 31 située en aval du module d'injection de NO, qui est éventuellement augmentée du volume de la chambre d'humidification lorsqu'elle est présente, permet de lisser les variations de concentration de NO inhalé par le patient et de se rapprocher de la valeur cible souhaitée, c'est-à-dire la posologie en NO.

Dans tous les cas, pouvoir s'approcher de la valeur cible de NO souhaitée grâce au système de dosage de secours 200 de NO intégré au dispositif de délivrance 1 de NO de l'invention améliore considérablement la sécurité pour le patient en comparaison avec un débit de NO de secours fixe (par exemple de 250 mL/min) usuellement délivré par le système de sécurité des dispositifs de délivrance de NO de l'art antérieur.

Ainsi, à titre comparatif, alors que le système de dosage de secours 200 de NO intégré au dispositif de délivrance 1 de NO de l'invention permet de garantir une concentration en NO sensiblement égale à la posologie souhaité, avec un système de secours basé sur un débit fixe de 250 mL/min, tel que classiquement mis en œuvre dans les dispositifs de délivrance de NO de l'art antérieur :
- pour un débit moyen de NO nécessaire de 0.05 L/min pour assurer normalement une concentration en NO de 10 ppmv (cas d'utilisation en néonatologie avec ventilateur de type HFO), la concentration résultante avec le débit fixe de 250 mL/min, est de 50 ppmv, ce qui correspond à une multiplication par 5 de la posologie souhaitée.
- à l'inverse, pour un débit moyen nécessaire de NO de 1 L/min pour assurer 80 ppmv de concentration en NO (cas d'utilisation chez l'adulte, par exemple en cas d'hypertension pulmonaire pendant une chirurgie cardiaque), la concentration résultante chute à 20 ppmv, ce qui correspond à une diminution de 75% de la posologie souhaitée.

Dans les deux cas, les écarts importants de posologie peuvent induire des situations inacceptables et dangereuses pour le patient et ce, contrairement au système de dosage de secours 200 de NO intégré au dispositif de délivrance 1 de NO de l'invention qui permet lui de respecter la posologie désirée.

Autrement dit, le système de dosage de secours 200 de NO de l'invention présente des avantages indéniables en renforçant la sécurité des patients en :
- injectant de façon automatique un débit de secours de NO sans attendre que l'utilisateur ne se rende compte de la situation et intervienne en basculant sur le dosage pneumatique de secours.
- garantissant que la concentration de NO inhalé par le patient est similaire à la concentration souhaitée par le médecin, c'est-à-dire la posologie désirée.

Bien entendu, le basculement sur le système de dosage de secours 200 de NO de l'invention n'est que temporaire, c'est-à-dire ne dure que le temps nécessaire au remplacement de l'équipement ou composant défaillant qui a déclenché le système d'alarme sonore et/ou visuel afin d'alerter le personnel soignant.

Afin d'éviter l'activation à mauvais escient du système de dosage de secours 200 de NO, l'unité de pilotage 130 est en outre configurée pour procéder à des séquences d'initialisation et d'extinction adéquates. Par exemple, en cas d'arrêt voulu par l'utilisateur de la thérapie de NO, l'unité de pilotage 130 peut commander l'actionneur 203 afin de fermer l'orifice calibré 204. Ainsi, en cas d'extinction volontaire et donc d'ouverture de l'électrovanne 202, la configuration « fermée » de l'orifice calibré 204 interdit alors toute circulation de débit de NO dans la ligne de secours 201, le temps que le dispositif de délivrance de NO 1 soit à l'arrêt.

D'une façon générale, selon l'invention, en cas de défaillance du capteur de débit 100 mesurant le débit de gaz issu du ventilateur mécanique 2 accompagnée d'une perte du signal de mesure, par exemple en cas de déconnexion des lignes amont 103 et/ou aval 102 de mesure de pression reliées au module de mesure de débit 100-1 du capteur de débit 100, l'unité de pilotage 130 conserve sa capacité à commander les actionneurs du dispositif de délivrance de NO 1.

Dans ce cas, l'unité de pilotage 130 peut détecter la perte de signal du capteur de débit 100 et alors immédiatement « activer » le système de dosage de secours 200 en stoppant le pilotage de que l'électrovanne proportionnelle 113 et de l'électrovanne 202 de sorte que l'électrovanne proportionnelle 113 passe en position de repos, c'est-à-dire en position fermée, et que l'électrovanne 202 de la ligne de secours 201 passe simultanément dans sa position de repos, à savoir une position ouverte autorisant une circulation du gaz provenant de la source de NO dans la ligne de secours 201 et au travers du dispositif de contrôle de débit 210 qui délivre alors le flux NO/N₂ au débit préréglé, comme expliqué ci-avant.

Le flux de mélange NO/N₂ peut dès lors emprunter la ligne de secours 201 au débit préfixé par le dispositif de contrôle de débit, par exemple un orifice calibré ou analogue, avant toute perte de signal due à un dysfonctionnement, à savoir le débit de secours déterminé par l'unité de pilotage 130 à partir de la dernière mesure de débit du gaz respiratoire obtenue avant dysfonctionnement et de la posologie souhaitée, c'est-à-dire de la teneur en NO désirée après mélange du flux de NO/N₂ avec le gaz respiratoire provenant du ventilateur 2, tel de l'air ou un mélange N₂/O₂, ledit mélange se faisant dans la branche inspiratoire 31 au niveau du module d'injection de NO 110, de sorte d'obtenir le mélange final à administrer au patient contenant la posologie en NO désirée, typiquement entre 1 et 80 ppmv, par exemple de l'ordre de 10 à 20 ppmv.

Autrement dit, le débit de NO/N₂ provenant de la ligne de secours 201, va ensuite rejoindre la ligne d'injection 111 (en 111b) pour être injecté dans la branche inspiratoire 31 du circuit patient 3 via le module d'injection de NO 110, et s'y mélanger avec le flux de gaz respiratoire, tel de l'air ou un mélange azote/oxygène, contenant de l'oxygène, typiquement environ au moins 21%vol. d'oxygène, provenant du ventilateur 2, comme déjà expliqué.

Bien entendu, une telle situation dans laquelle l'unité de pilotage 130 détecte une perte de signal du capteur de débit 100, peut se résoudre et retourner ensuite à la normale, par exemple lorsque l'utilisateur reconnecte les lignes amont 103 et/ou ligne aval 102 de mesure de pression dudit capteur de débit 100, si elle(s) a/ont été déconnectée(s) involontairement de sorte d'engendrer le dysfonctionnement et la perte de signal.

Ensuite, lorsque l'unité de pilotage 130 détermine que le signal du capteur de débit 100 est de nouveau valide, i.e. restauration de la réception du signal, elle peut revenir dans un état de fonctionnement normal du dispositif de délivrance de NO 1, c'est-à-dire en commandant à nouveau l'électrovanne 202 dans une position fermée pour isoler le système de dosage de secours 200 de la ligne d'injection 111, et par ailleurs en pilotant à nouveau l'électrovanne proportionnelle 113 afin de délivrer le débit de NO devant être injecté dans la ligne d'injection 111 et le module d'injection de NO 110 afin de satisfaire la concentration de NO souhaitée dans le gaz fourni au patient.

De manière analogue, bien que cette occurrence soit plus rare, l'unité de pilotage 130 peut par ailleurs déterminer qu'une défaillance interne du capteur de débit de NO 112 est apparue, telle qu'une rupture de son câble d'alimentation, occasionnée par des vibrations dans le cadre d'un transport de patient par exemple.

Dans ce cas, l'unité de pilotage 130 est configurée pour opérer de façon identique au cas précédent résultant d'une perte de signal du capteur de débit 100 du fait d'un débranchement accidentel des lignes amont 103 et/ou ligne aval 102, afin d'activer le système de dosage de secours 200. Toutefois, une telle défaillance interne du capteur 112 est généralement permanente et irréversible, et nécessite donc une intervention technique pour remplacer les éléments détériorés, i.e. dysfonctionnant.

Le dispositif de délivrance de NO 1 équipé du système de dosage de secours 200 de NO de l'invention est particulièrement bien adaptée à la fourniture de mélange gazeux comprenant de 1 à 80 ppmv de NO (posologie) et au moins 21%vol. d'oxygène à des patients (adultes, enfants, adolescents ou nouveau-nés), souffrant d'hypertensions pulmonaires et/ou d'hypoxie, qui peuvent engendrer des vasoconstrictions pulmonaires ou analogues, par exemple causés par des pathologies ou troubles pulmonaires de type PPHN (hypertension pulmonaire persistante du nouveau-né) ou SDRA (syndrome de détresse respiratoire aigüe), ou encore engendrés par une opération de chirurgie cardiaque avec mise du patient sous circulation sanguine extracorporelle.

## Revendications

1. Installation de fourniture de gaz (1, 2) à un patient comprenant :
- un dispositif de délivrance de NO (1) configuré pour fournir un mélange gazeux NO/N₂ comprenant :
▪ une ligne d'injection de NO (111) pour acheminer le mélange gazeux NO/N₂,
▪ un dispositif à vanne (113) agencé sur la ligne d'injection (111) pour contrôler la circulation du mélange gazeux NO/N₂ dans la ligne d'injection (111),
▪ une ligne de secours (201) venant se raccorder fluidiquement à la ligne d'injection (111) en amont et en aval du dispositif à vanne (113), ladite ligne de secours (201) comprenant une électrovanne de secours (202) et un dispositif de contrôle de débit (210), et
▪ des moyens de pilotage (130) configurés pour coopérer avec l'électrovanne de secours (202), le dispositif de contrôle de débit (210) et le dispositif à vanne (113),
- un ventilateur médical (2) configuré pour fournir un gaz respiratoire contenant de l'oxygène,
- un circuit patient (3) auquel sont raccordés fluidiquement le dispositif de délivrance de NO (1) et le ventilateur médical (2) pour alimenter ledit circuit patient (3) en ledit mélange gazeux NO/N₂ et en ledit gaz respiratoire contenant de l'oxygène, et
- un capteur de débit (100) configuré pour fournir aux moyens de pilotage (130) du dispositif de délivrance de NO (1), au moins un signal de mesure représentatif d'au moins un débit gazeux au sein du circuit patient (3), et dans lequel en cas d'interruption de la réception, par les moyens de pilotage (130) du dispositif de délivrance de NO (1), dudit au moins un signal de mesure fourni par le capteur de débit (100) :
- l'électrovanne de secours (202) est configurée pour passer en position ouverte pour permettre une circulation du mélange gazeux NO/N₂ dans la ligne de secours (201),
- le dispositif à vanne (113) est configuré pour passer en position fermée pour stopper toute circulation de gaz dans la ligne d'injection (111), et
- le dispositif de contrôle de débit (210) est configuré pour fournir le mélange gazeux NO/N₂ à un débit gazeux de secours préfixé, où ledit débit gazeux de secours a été déterminé par les moyens de pilotage (130) à partir d'au moins un signal de mesure fourni par le capteur de débit (100), avant ladite interruption de réception dudit signal, et préréglé par commande du dispositif de contrôle de débit (210) par lesdits moyens de pilotage (130), avant ladite interruption de réception dudit signal.

2. Installation selon la revendication 1, **caractérisée en ce que** le capteur de débit (100) est agencé sur le circuit patient (3).

3. Installation selon l'une des revendications précédentes, **caractérisée en ce que** le capteur de débit (100) est un capteur de débit massique ou à mesure de pression différentielle.

4. Installation selon l'une des revendications précédentes, **caractérisée en ce que** le capteur de débit (100) est du type à mesure de pression différentielle comprenant un module de mesure (100-1) traversé par un passage interne de gaz comprenant une restriction interne (101), une ligne amont (103) et une ligne aval (102) de mesure de pression étant connectées fluidiquement au module de mesure (100-1) du capteur de débit (100) de manière à permettre de mesurer la pression, en amont et en aval de la restriction interne (101).

5. Installation selon la revendication 4, **caractérisée en ce qu'**un capteur de pression différentiel (104) est raccordé au capteur de débit (100) via les lignes amont (103) et aval (102) de mesure de pression, ledit capteur de pression différentiel (104) étant agencé dans le dispositif de délivrance de NO (1) et coopérant avec les moyens de pilotage (130) pour leur fournir les valeurs de pression mesurées par le capteur de débit (100).

6. Installation selon l'une des revendications précédentes, **caractérisée en ce que** les moyens de pilotage (130) sont configurés pour déterminer le débit gazeux de secours à partir d'au moins un signal de mesure fourni par le capteur de débit (100), avant ladite interruption de réception dudit signal, et d'une concentration de NO finale désirée.

7. Installation selon les revendications 1 et 6, **caractérisée en ce que** le débit gazeux de secours est déterminé à partir d'un débit de gaz moyen calculé à partir de plusieurs débits de gaz mesurés par le capteur de débit (100) pendant une durée donnée, avant ladite interruption de réception dudit signal, de préférence la durée donné est comprise entre plusieurs secondes et plusieurs dizaines de secondes.

8. Installation selon l'une des revendications précédentes, **caractérisée en ce que** le dispositif à vanne (113) comprend une électrovanne proportionnelle.

9. Installation selon les revendications 1 ou 6, **caractérisée en ce que** les moyens de pilotage (130) sont configurés pour déterminer un débit de secours à partir d'au moins un débit de gaz respiratoire mesuré par le capteur de débit (100), avant l'interruption de réception dudit signal, et d'une concentration de NO finale à obtenir après mélange du mélange NO/azote provenant du dispositif de délivrance de NO (1) et du flux de gaz respiratoire contenant de l'O₂ provenant du ventilateur (2), typiquement de l'air ou un mélange oxygène/azote.

10. Installation selon la revendication 1, **caractérisée en ce que** le dispositif de contrôle de débit (210) comprend un système à orifice calibré proportionnel commandé par les moyens de pilotage (130) pour régler le débit gazeux de secours préfixé.

11. Installation selon la revendication 1, **caractérisée en ce que** les moyens de pilotage (130) du dispositif de délivrance de NO (1) comprennent au moins un microprocesseur.

12. Installation selon la revendication 1, **caractérisée en ce que** le dispositif à vanne (113) du dispositif de délivrance de NO (1) est configuré pour être normalement dans une position fermée correspondant à un état repos, empêchant toute circulation de gaz dans la ligne d'injection (111).

13. Installation selon la revendication 1, **caractérisée en ce que** l'électrovanne de secours (202) du dispositif de délivrance de NO (1) est configurée pour être normalement dans une position ouverte correspondant à un état de repos permettant une circulation de gaz dans la ligne de secours (201).

14. Installation selon les revendications 1, 12 et 13 **caractérisée en ce qu'**en cas de perte de signal, les moyens de pilotage (130) sont configurés pour arrêter de piloter le dispositif à vanne (113) et l'électrovanne de secours (202) du dispositif de délivrance de NO (1) qui passent alors automatiquement dans leur état de repos.

15. Installation selon la revendication 1, **caractérisée en ce que** les moyens de pilotage (130) du dispositif de délivrance de NO (1) sont en outre configurés pour, préalablement à toute interruption de la réception ou perte de signal provenant du capteur de débit (100), agir sur un système à orifice calibré proportionnel () du dispositif de contrôle de débit (210) de la ligne de secours (201) pour prérégler le débit gazeux de secours désiré.

## Patentansprüche

1. Anlage zur Bereitstellung von Gas (1, 2) für einen Patienten, umfassend:
- eine NO-Abgabevorrichtung (1), die dazu ausgestaltet ist, ein NO/N₂-Gasgemisch bereitzustellen, umfassend:
■ eine NO-Zuführungsleitung (111), um das NO/N₂-Gasgemisch weiterzuleiten,
■ eine Ventilvorrichtung (113), die an der Zuführungsleitung (111) angeordnet ist, um die Zirkulation des NO/N₂-Gasgemisches in der Zuführungsleitung (111) zu regeln,
■ eine Ersatzleitung (201), die stromauf und stromab der Ventilvorrichtung (113) an die Zuführungsleitung (111) angeschlossen ist, wobei die Ersatzleitung (201) ein Ersatzmagnetventil (202) und eine Durchflussmengenregelungsvorrichtung (210) umfasst, und
■ Ansteuerungsmittel (130), die dazu ausgestaltet sind, mit dem Ersatzmagnetventil (202), der Durchflussmengenregelungsvorrichtung (210) und der Ventilvorrichtung (113) zusammenzuwirken,
- ein medizinisches Beatmungsgerät (2), das dazu ausgestaltet ist, ein Sauerstoff enthaltendes Atemgas bereitzustellen,
- einen Patientenkreislauf (3), an den die NO-Abgabevorrichtung (1) und das medizinische Beatmungsgerät (2) fluidisch angeschlossen sind, um den Patientenkreislauf (3) mit dem NO/N₂-Gasgemisch und mit dem Sauerstoff enthaltenden Atemgas zu versorgen, und
- einen Durchflussmengensensor (100), der dazu ausgestaltet ist, den Ansteuerungsmitteln (130) der NO-Abgabevorrichtung (1) mindestens ein Messsignal bereitzustellen, das für mindestens eine Gasdurchflussmenge in dem Patientenkreislauf (3) repräsentativ ist, und bei der im Fall einer Unterbrechung des Empfangs, durch die Ansteuerungsmittel (130) der NO-Abgabevorrichtung (1), des mindestens einen von dem Durchflussmengensensor (100) bereitgestellten Messsignals:
- das Ersatzmagnetventil (202) dazu ausgestaltet ist, in eine geöffnete Stellung überzugehen, um eine Zirkulation des NO/N₂-Gasgemisches in der Ersatzleitung (201) zu ermöglichen,
- die Ventilvorrichtung (113) dazu ausgestaltet ist, in eine geschlossene Stellung überzugehen, um jede Zirkulation von Gas in der Zuführungsleitung (111) zu stoppen, und
- die Durchflussmengenregelungsvorrichtung (210) dazu ausgestaltet ist, das NO/N₂-Gasgemisch mit einer vorgegebenen Ersatzgasdurchflussmenge bereitzustellen, wobei die Ersatzgasdurchflussmenge von den Ansteuerungsmitteln (130) ausgehend von mindestens einem von dem Durchflussmengensensor (100) bereitgestellten Messsignal vor der Empfangsunterbrechung des Signals bestimmt und durch Steuern der Durchflussmengenregelungsvorrichtung (210) durch die Ansteuerungsmittel (130) vor der Empfangsunterbrechung des Signals voreingestellt worden ist.

2. Anlage nach Anspruch 1, **dadurch gekennzeichnet, dass** der Durchflussmengensensor (100) an dem Patientenkreislauf (3) angeordnet ist.

3. Anlage nach einem der vorhergehenden Ansprüche, **dadurch gekennzeichnet, dass** der Durchflussmengensensor (100) ein Massendurchflusssensor oder ein Differenzdruckmesssensor ist.

4. Anlage nach einem der vorhergehenden Ansprüche, **dadurch gekennzeichnet, dass** der Durchflussmengensensor (100) vom Typ mit Differenzdruckmessung ist, umfassend ein Messmodul (100-1), das von einem internen Gasdurchlass (101) durchquert wird, der eine interne Verengung umfasst, wobei eine stromaufwärtige Leitung (103) und eine stromabwärtige Leitung (102) zur Druckmessung fluidisch an das Messmodul (100-1) des Durchflussmengensensors (100) angeschaltet sind, so dass das Messen des Drucks stromauf und stromab der internen Verengung (101) ermöglicht wird.

5. Anlage nach Anspruch 4, **dadurch gekennzeichnet, dass** ein Differenzdrucksensor (104) an den Durchflussmengensensor (100) über die stromaufwärtigen (103) und stromabwärtigen (102) Leitungen zur Druckmessung angeschlossen ist, wobei der Differenzdrucksensor (104) in der NO-Abgabevorrichtung (1) angeordnet ist und mit den Ansteuerungsmitteln (130) zusammenwirkt, um ihnen die von dem Durchflussmengensensor (100) gemessenen Druckwerte bereitzustellen.

6. Anlage nach einem der vorhergehenden Ansprüche, **dadurch gekennzeichnet, dass** die Ansteuerungsmittel (130) dazu ausgestaltet sind, die Ersatzgasdurchflussmenge ausgehend von mindestens einem von dem Durchflussmengensensor (100) vor der Empfangsunterbrechung des Signals bereitgestellten Messsignal und von einer gewünschten finalen NO-Konzentration zu bestimmen.

7. Anlage nach den Ansprüchen 1 und 6, **dadurch gekennzeichnet, dass** die Ersatzgasdurchflussmenge ausgehend von einer mittleren Gasdurchflussmenge bestimmt wird, die ausgehend von mehreren von dem Durchflussmengensensor (100) während einer gegebenen Dauer vor der Empfangsunterbrechung des Signals gemessenen Gasdurchflussmengen berechnet wird, wobei die gegebene Dauer bevorzugt zwischen mehreren Sekunden und mehreren zehn Sekunden beträgt.

8. Anlage nach einem der vorhergehenden Ansprüche, **dadurch gekennzeichnet, dass** die Ventilvorrichtung (113) ein Proportionalmagnetventil umfasst.

9. Anlage nach den Ansprüchen 1 oder 6, **dadurch gekennzeichnet, dass** die Ansteuerungsmittel (130) dazu ausgestaltet sind, eine Ersatzdurchflussmenge ausgehend von mindestens einer Atemgasdurchflussmenge, die von dem Durchflussmengensensor (100) vor der Empfangsunterbrechung des Signals gemessen wird, und von einer finalen NO-Konzentration, die nach dem Mischen des von der NO-Abgabevorrichtung (1) kommenden NO/Stickstoff-Gemisches und des von dem Beatmungsgerät (2) kommenden O₂ enthaltenden Atemgasstroms, typischerweise Luft oder ein Sauerstoff/StickstoffGemisch, zu erhalten ist, zu bestimmen.

10. Anlage nach Anspruch 1, **dadurch gekennzeichnet, dass** die Durchflussmengenregelungsvorrichtung (210) ein System mit proportionaler kalibrierter Bohrung umfasst, das durch die Ansteuerungsmittel (130) gesteuert wird, um die vorgegebene Ersatzgasdurchflussmenge einzustellen.

11. Anlage nach Anspruch 1, **dadurch gekennzeichnet, dass** die Ansteuerungsmittel (130) der NO-Abgabevorrichtung (1) mindestens einen Mikroprozessor umfassen.

12. Anlage nach Anspruch 1, **dadurch gekennzeichnet, dass** die Ventilvorrichtung (113) der NO-Abgabevorrichtung (1) dazu ausgestaltet ist, normalerweise in einer geschlossenen Stellung zu sein, die einem Ruhezustand entspricht und jede Zirkulation von Gas in der Zuführungsleitung (111) verhindert.

13. Anlage nach Anspruch 1, **dadurch gekennzeichnet, dass** das Ersatzmagnetventil (202) der NO-Abgabevorrichtung (1) dazu ausgestaltet ist, normalerweise in einer geöffneten Stellung zu sein, die einem Ruhezustand entspricht und eine Zirkulation von Gas in der Ersatzleitung (201) ermöglicht.

14. Anlage nach den Ansprüchen 1, 12 und 13, **dadurch gekennzeichnet, dass** im Fall eines Signalverlustes die Ansteuerungsmittel (130) dazu ausgestaltet sind, das Ansteuern der Ventilvorrichtung (113) und des Ersatzmagnetventils (202) der NO-Abgabevorrichtung (1) zu stoppen, die daraufhin automatisch in ihren Ruhezustand übergehen.

15. Anlage nach Anspruch 1, **dadurch gekennzeichnet, dass** die Ansteuerungsmittel (130) der NO-Abgabevorrichtung (1) ferner dazu ausgestaltet sind, vor jeder Unterbrechung des Empfangs oder einem Verlust eines von dem Durchflussmengensensor (100) kommenden Signals auf ein System mit proportionaler kalibrierter Bohrung () der Durchflussmengenregelungsvorrichtung (210) der Ersatzleitung (201) einzuwirken, um die gewünschte Ersatzgasdurchflussmenge voreinzustellen.

## Claims

1. Installation (1, 2) for supplying gas to a patient, comprising:
- an NO delivery device (1) configured to supply an NO/N₂ gas mixture, comprising:
■ an NO injection line (111) for conveying the NO/N₂ gas mixture,
■ a valve device (113) arranged on the injection line (111) in order to control the circulation of the NO/N₂ gas mixture in the injection line (111),
■ a backup line (201) that connects fluidly to the injection line (111) upstream and downstream of the valve device (113), said backup line (201) comprising a backup solenoid valve (202) and a flow rate control device (210), and
■ operating means (130) configured to interact with the backup solenoid valve (202), the flow rate control device (210) and the valve device (113),
- a medical ventilator (2) configured to supply a respiratory gas containing oxygen,
- a patient circuit (3) to which the NO delivery device (1) and the medical ventilator (2) are fluidly connected in order to supply said patient circuit (3) with said NO/N₂ gas mixture and with said respiratory gas containing oxygen, and
- a flow rate sensor (100) configured to supply to the operating means (130) of the NO delivery device (1) at least one measurement signal representing at least one gas flow rate within the patient circuit (3),
and in which in the event of the interruption of reception, by the operating means (130) of the delivery device (1), of said at least one measurement signal supplied by the flow rate sensor (100):
- the backup solenoid valve (202) is configured to switch to an open position in order to allow the circulation of the NO/N₂ gas mixture in the backup line (201),
- the valve device (113) is configured to switch to a closed position in order to stop any circulation of gas in the injection line (111), and
- the flow rate control device (210) is configured to supply the NO/N₂ gas mixture at a pre-set backup gas flow rate, where said backup gas flow rate has been determined by the operating means (130) on the basis of at least one measurement signal supplied by the flow rate sensor (100), before said interruption of reception of said signal, and pre-regulated by a command to the flow rate control device (210) from said operating means (130), before said interruption of reception of said signal.

2. Installation according to Claim 1, **characterized in that** the flow rate sensor (100) is arranged on the patient circuit (3).

3. Installation according to one of the preceding claims, **characterized in that** the flow rate sensor (100) is a mass flow sensor or a differential pressure measurement sensor.

4. Installation according to one of the preceding claims, **characterized in that** the flow rate sensor (100) is of the type that measures differential pressure, comprising a measurement module (100-1) traversed by an internal gas passage comprising an internal restriction (101), an upstream line (103) and a downstream line (102) for measuring pressure being fluidly connected to the measurement module (100-1) of the flow rate sensor (100) so as to make it possible to measure the pressure upstream and downstream of the internal restriction (101).

5. Installation according to Claim 4, **characterized in that** a differential pressure sensor (104) is connected to the flow rate sensor (100) via the upstream (103) and downstream (102) pressure measurement lines, said differential pressure sensor (104) being arranged in the delivery device (1) and interacting with the operating means (130) in order to supply them with the pressure values measured by the flow rate sensor (100).

6. Installation according to one of the preceding claims, **characterized in that** the operating means (130) are configured to determine the backup gas flow rate on the basis of at least one measurement signal supplied by the flow rate sensor (100), before said interruption of reception of said signal, and on the basis of a desired final concentration.

7. Installation according to Claims 1 and 6, **characterized in that** the backup gas flow rate is determined on the basis of an average gas flow rate calculated on the basis of a plurality of gas flow rates measured by the flow rate sensor (100) in a given duration, before said interruption of reception of said signal; the given duration is preferably between several seconds and several tens of seconds.

8. Installation according to one of the preceding claims, **characterized in that** the valve device (113) comprises a proportional solenoid valve.

9. Installation according to Claim 1 or 6, **characterized in that** the operating means (130) are configured to determine a backup flow rate on the basis of at least one respiratory gas flow rate measured by the flow rate sensor (100), before the interruption of reception of said signal, and on the basis of a final NO concentration to be obtained after mixing of the NO/nitrogen mixture coming from the NO delivery device (1) and the flow of respiratory gas containing O₂ coming from the ventilator (2), typically air or an oxygen/nitrogen mixture.

10. Installation according to Claim 1, **characterized in that** the flow rate control device (210) comprises a proportional calibrated orifice system commanded by the operating means (130) to regulate the pre-set backup gas flow rate.

11. Installation according to Claim 1, **characterized in that** the operating means (130) of the NO delivery device (1) comprise at least one microprocessor.

12. Installation according to Claim 1, **characterized in that** the valve device (113) of the NO delivery device (1) is configured so that it is normally in a closed position corresponding to an idle state, preventing any circulation of gas in the injection line (111).

13. Installation according to Claim 1, **characterized in that** the backup solenoid valve (202) of the NO delivery device (1) is configured so that it is normally in an open position corresponding to an idle state, allowing the circulation of gas in the backup line (201).

14. Installation according to Claims 1, 12 and 13, **characterized in that** in the event of a loss of signal, the operating means (130) are configured to stop operating the valve device (113) and the backup solenoid valve (202) of the NO delivery device (1), which then automatically switch to their idle state.

15. Installation according to Claim 1, **characterized in that** the operating means (130) of the NO delivery device (1) are further configured to, prior to any interruption of the reception or loss of the signal coming from the flow rate sensor (100), act on a proportional calibrated orifice system () of the flow rate control device (210) of the backup line (201) in order to pre-regulate the desired backup gas flow rate.
